# EUROPEAN PATENT APPLICATION

(11) **EP 4 610 280 A1**
(43) Date of publication of application: **03.09.2025**
(21) Application number: 23881349.7
(22) Date of filing: 24.07.2023
(51) Int. Cl.: C07K 19/00, C12N 9/02, C12N 15/70, C12N 1/21, C12R 1/19

(54) **METHOD FOR PRODUCING POLYPEPTIDE FROM RECOMBINANT FUSION PROTEIN AND USE THEREOF**

(30) Priority: 24.10.2022 CN 202211305495
(71) Applicant: Yangzhou Aurisco Pharmaceutical Co., Ltd, Yangzhou, Jiangsu 225100 (CN); Guangdong Zeta Pharmaceutical Co., Ltd., Qingyuan, Guangdong 511500 (CN)
(72) Inventor: LI, Yan, Qingyuan, Guangdong 511500 (CN); LI, Jieping, Qingyuan, Guangdong 511500 (CN); HUANG, Meiqun, Qingyuan, Guangdong 511500 (CN)
(74) Representative: Mewburn Ellis LLP
(86) International application number: PCT/CN2023/108890
(87) International publication number: WO 2024/087761

(57) **Abstract**

The present invention relates to a method for producing a polypeptide from a recombinant fusion protein, and disclosed are a method for producing a polypeptide by means of serially combining multiple protease cleavage sites and polypeptide sequences in a specific order under the assistance of protein chaperones, and the use thereof. The high-tandem number recombinant engineering bacteria constructed in the present invention can efficiently express soluble recombinant proteins without forming inclusion bodies and with less impurity proteins. The fusion protein captured after high-pressure crushing can be quickly digested by an enzyme within 1-2 h to obtain a target polypeptide monomer. The expression system can be used to produce various polypeptides having 10-60 amino acids and/or an isoelectric point range of 2-10. Compared with a single serial connection, the yield of the target polypeptide is greatly improved. The method is more suitable for industrial large-scale production.

## Description

### FIELD OF THE INVENTION

The present invention relates to the field of biotechnology. In particular, the present invention relates to a method for producing a polypeptide from a recombinant fusion protein and uses thereof.

### BACKGROUND OF THE INVENTION

Polypeptides are a type of compound formed by connecting amino acids together in the form of peptide bonds. Polypeptides are commonly found in living organisms, and tens of thousands of different types of peptides have been discovered so far. They widely participate in and regulate the functional activities of various systems, organs, tissues, and cells in the body, playing an important role in life activities. In recent years, peptide drugs have maintained good development prospects as a key area of innovative research and development in biopharmaceuticals both domestically and internationally. Although peptide drugs have a relatively small market share in the global biopharmaceutical market, they are developing rapidly. In 2019, the global market size of polypeptide drugs exceeded 30 billion US dollars, with an average annual compound growth rate of around 10%.

With the deepening of proteomics research, the requirements for polypeptide synthesis are becoming increasingly high, and the synthesized peptide sequences are becoming longer, which puts higher demands on peptide synthesis technology. Common peptide drugs can be naturally extracted, chemically synthesized, or obtained through genetic engineering methods.

The polypeptide involved in the invention is a human glucagon like peptide-1 (GLP-1) analog, which is a weekly preparation developed by Novo Nordisk that can significantly improve the blood sugar level of patients with type 2 diabetes. The chemical synthesis of this peptide usually adopts solid-phase coupling condensation method, which has relatively low reaction efficiency, high purification difficulty, and high cost.

Genetic engineering methods generally construct hybrid DNA molecules *in vitro,* and then introduce them into live cells to alter the original genetic characteristics of organisms, obtain new varieties, and produce new products. Microbial expression systems have the advantages of simple cultivation, short periodicity, and low cost, making them an important source of commercial protein expression. Commonly used microbial expression hosts include *Escherichia coli, Bacillus subtilis, yeast,* etc. The expression products of prokaryotic expression are highly efficient, relatively stable, not easily degraded by the host, and due to the short proliferation time and low cultivation cost of *Escherichia coli,* the E. coli expression system has become a relatively quick way to obtain heterologous proteins.

Expression of fusion proteins is a commonly used strategy in protein expression, in which the coding regions of two or more genes are connected end-to-end and controlled by the same regulatory sequence, resulting in high efficiency, minimal degradation, and simple and convenient purification of the target protein fusion expression. In addition, chemical reagent cleavage sites or protease cleavage sites constructed in fusion expression vectors can be used to remove the prokaryotic peptide segments of the fusion protein *in vitro,* thereby obtaining natural protein products. The currently widely used fusion expression systems include PA system, PG system, His fusion system, GST system, FLAG system, MBP system, TrxA fusion system, etc. Fusion expression can not only increase protein expression levels, but also help target proteins fold correctly, thereby obtaining soluble proteins. Usually, in order to fully utilize the potential of the host strain, multiple target proteins are expressed in tandem. However, as the number of tandem target proteins increases, the molecular weight of the fusion protein increases, and it becomes increasingly difficult to meet the needs of high soluble and high expression levels of high-tandem fusion proteins.

The prior art CN101910193A disclosed a preparation method of GLP-1 analogues, which uses *Saccharomyces cerevisiae* as the expression host and provides a production method of biosynthetic GLP-1 analogue peptides. The disadvantages are: (1) The fermentation period of yeast itself is long, and the cost is higher than that of *Escherichia coli;* (2) Although the expression system of GLP-1 analog peptides has been optimized to make the Kex2 protease of *Saccharomyces cerevisiae* itself more easily cleaved to obtain GLP-1 analogue peptides, the expression of each analog peptide is inserted into the expression vector in the form of a single peptide sequence, thus limiting the potential of the host for expression. (3) The GLP-1 analog peptides produced after enzymatic cleavage by the Kex2 protease released by yeast itself inevitably have protrusions at the N-terminal and C-terminal ends, requiring additional enzymatic cleavage treatment.

Using *Escherichia coli* as the expression host, the prior art CN110128521A disclosed an auxiliary protein for producing recombinant fusion proteins, in which the auxiliary protein is tandemly connected with the enzymatic cleavage site of enterokinase and the GLP-1 analogue. Although soluble fusion proteins are expressed in this technology, only one target peptide unit is included in the fusion protein. As the number of tandem target polypeptides increases, auxiliary proteins need to exhibit both the functions of promoting expression and facilitating solubility. Some auxiliary proteins cannot meet the requirements for soluble expression of high-tandem fusion proteins. For multiple tandem target polypeptides, it is still necessary to develop soluble expression methods that can ensure high-level expression.

The prior art CN110305223A disclosed a fusion protein including multiple target protein sequences in tandem. The fusion protein generates multiple free target proteins under the action of Kex2 and CPB proteases, achieving tandem expression of four GLP-1 analogues. However, the connection between two adjacent GLP-1 analogue polypeptides depends only on "KR", and the fusion protein with only 4 "KR" cleavage sites requires overnight enzymatic cleavage to obtain free target proteins, seriously affecting the enzymatic cleavage rate. In addition, the fusion protein in this technology contains the promoting peptide "EEAEAEARG", however a great amount of insoluble fusion proteins will still be produced.

Therefore, there is a need in this field to develop a convenient and efficient production system for polypeptide sequence.

### SUMMARY OF THE INVENTION

The purpose of the present invention is to provide a method for producing a polypeptide from a recombinant fusion protein and uses thereof.

In the first aspect of the present invention, a recombinant fusion protein is provided, wherein the recombinant fusion protein comprises interconnected:
a) a protein chaperone, wherein said protein chaperone is thioredoxin; b) n target polypeptides in tandem;
c) a protease recognition sequence, wherein said protease-recognition sequence is used to cleave the recombinant fusion protein into n independent target polypeptides by protease;
n represents an integer of 2 - 20, preferably 3 - 15, and more preferably 5 - 10.

In another preferred embodiment, the recombinant fusion protein has the following sequentially connected structures from the N-terminus to C-terminus:

Protein chaperone-{N-terminal protease recognition sequence-target polypeptide-C-terminal protease recognition sequence-linker peptide}ₙ₋₁-N-terminal protease recognition sequence-target polypeptide.

In another preferred embodiment, the thioredoxin is derived from *thermophilic bacteria,* preferably from the *Pyrodictium occultum, Hyperthermus butylicus, Pyrolobus fumarii,* or *Aeropyrum pernix K1.*

In another preferred embodiment, the thioredoxin is selected from the groups consisting of:
(1) an amino acid sequence of SEQ ID NO: 8, SEQ ID NO: 10, SEQ ID NO: 12, SEQ ID NO: 14, SEQ ID NO: 16 or SEQ ID NO: 18;
(2) a polypeptide having at least 80%, at least 85%, or at least 90%, 93%, 95%, 96%, 97%, 98%, 99% amino acid sequence homolog with SEQ ID NO: 8, SEQ ID NO: 10, SEQ ID NO: 12, SEQ ID NO: 14, SEQ ID NO: 16 or SEQ ID NO: 18 and having the function of promoting the solubility of the fusion protein.

In another preferred embodiment, the N-terminal protease recognition sequence is a sequence that can be recognized and cleaved by a protease selected from the group consisting of enterokinase, thrombin, SUMO protease, TEV protease, Factor Xa protease, or a combination thereof.

In another preferred embodiment, the N-terminal protease recognition sequence having an amino acid sequence of SEQ ID NO: 1, SEQ ID NO: 2, SEQ ID NO: 3, SEQ ID NO: 4, or SEQ ID NO: 5.

In another preferred embodiment, the C-terminal protease recognition sequence is a sequence that can be recognized and cleaved by a protease selected from the group consisting of Kex2 protease, carboxypeptidase B (CPB), or a dual-enzyme recognition sequence formed by their tandem connection.

In another preferred embodiment, the C-terminal protease recognition sequence is selected from the group: KR or RR.

In another preferred embodiment, the connecting peptide is a flexible connecting peptide.

In another preferred embodiment, the amino acid sequence of the linker peptide is selected from the group consisting of: GGGGS, GSGSG, GSGG, GSGS, GSG, or combinations thereof; preferably GSGSG.

In another preferred embodiment, the number of amino acids of the target polypeptide is 10 to 100, preferably 20 to 80, more preferably 20 to 60, and most preferably 30 to 40.

In another preferred embodiment, the isoelectric point range of the target polypeptide is 2-10.

In another preferred embodiment, the target polypeptide is selected from the group consisting of: glucagon-like peptide, parathyroid hormone, growth factor, collagen, or combinations thereof.

In another preferred embodiment, the target polypeptide is glucagon-like peptide-1 (GLP-1) or analogues thereof, glucagon-like peptide (GLP-2) or analogues thereof, gastric inhibitory polypeptide (GIP), peptide tyrosine tyrosine (PYY), parathyroid hormone (PTH), growth factors, collagen;

Preferably, glucagon-like peptide-1 (GLP-1) or analogues thereof, glucagon-like peptide (GLP-2) or analogues thereof, gastric inhibitory polypeptide (GIP), peptide tyrosine tyrosine (PYY), growth factors, collagen;

Most preferably, glucagon-like peptide-1 (GLP-1) or analogues thereof.

In another preferred embodiment, the recombinant fusion protein is soluble.

In another preferred embodiment, the recombinant fusion protein comprises amino acid sequences of SEQ ID NO: 20, SEQ ID NO: 22, SEQ ID NO: 24, SEQ ID NO: 26, SEQ ID NO: 28; and preferably, the recombinant fusion protein comprises amino acid sequences of SEQ ID NO: 20.

In the second aspect of the present invention, a polynucleotide encoding the fusion protein of the first aspect of the present invention is provided.

In another preferred embodiment, the polynucleotide comprises a gene encoding sequence of protein chaperones of SEQ ID NO: 9, SEQ ID NO: 11, SEQ ID NO: 13, SEQ ID NO: 15, SEQ ID NO: 17.

In another preferred embodiment, the polynucleotide comprises a nucleotide sequence of SEQ ID NO: 21, SEQ ID NO: 23, SEQ ID NO: 25, SEQ ID NO: 27, SEQ ID NO: 29.

In the third aspect of the present invention, a vector comprising the polynucleotide of the second aspect of the present invention is provided.

In another preferred embodiment, the vector comprises a gene encoding thioredoxin.

In another preferred embodiment, the vector is selected from the following group: pET series expression vectors, pQE series expression vectors, or a combination thereof.

In another preferred embodiment, the vector is pET32b(+) expression vectors, preferably, pET32b(+)-kan expression vectors.

In the fourth aspect of the present invention, an engineered host cell is provided, comprising the vector of the third aspect of the present invention, or having the polynucleotide of the second aspect of the present invention integrated into its genome.

In another preferred embodiment, the host cell is selected from the following group: *Escherichia coli, yeast,* or a combination thereof.

In another preferred embodiment, the host cell is *Escherichia coli* BL21(DE3).

In the fifth aspect of the present invention, a method for synthesizing a target polypeptide is provided, comprising:
S1) Culturing the engineered host cell of the fourth aspect of the present invention under a suitable condition to obtain the fusion protein of the first aspect of the present invention; and
S2) Using an enzyme that specifically cleaves the protease recognition sequence in the fusion protein obtained in step S1) to cleave the fusion protein, thereby obtaining a separate target polypeptide.

In another preferred embodiment, the step S1) comprises:
S1a) Inoculating the engineered host cell in a culture medium containing corresponding antibiotics for culturing, thereby obtaining the host cell bacterial liquid.
S1b) Inoculating the bacterial liquid and culture medium together in shake flasks containing corresponding antibiotics for culturing, and then adding a fermentation inducer to induce expression, thereby obtaining the fusion protein.

In another preferred embodiment, in the step S1a, the culture is carried out at 33-40°C, preferably 35-38°C, and more preferably 36-37°C.

In another preferred embodiment, in the step S1a, the rotational speed of the shake flasks is 180-260 rpm, preferably 200-240 rpm, and more preferably 210-220 rpm.

In another preferred embodiment, in the step S1a, the culture time is 1-48 hours, preferably 8-24 hours, and more preferably 15-16 hours.

In another preferred embodiment, in the step S1b, the bacterial liquid and culture medium inoculation are inoculated at 1:1-1000 (v:v), preferably 1:50-500, and more preferably 1: 100-200.

In another preferred embodiment, in the step S1b, the concentration of the antibiotic is 1-100 ug/ml, preferably 20-70 ug/ml, and more preferably 30-50 ug/ml.

In another preferred embodiment, in the step S1b, the culture is carried out at 33-40°C, preferably 35-38°C, and more preferably 36-37°C.

In another preferred embodiment, in the step S1b, the rotational speed of the shake flasks is 180-260 rpm, preferably 200-240 rpm, and more preferably 210-220 rpm.

In another preferred embodiment, in the step S1b, the fermentation inducer is added when the OD₆₀₀ value is about 0.4-1.2, preferably 0.8-1.

In another preferred embodiment, the fermentation inducer is IPTG.

In another preferred embodiment, the final concentration of the added fermentation inducer is 0.05-5 mM, preferably 0.1-3 mM, and more preferably 0.5-1 mM.

In another preferred embodiment, the induction is performed at 16-40°C, preferably 25-35°C, and more preferably 28-30°C.

In another preferred embodiment, the induction time is 1-48 hours, preferably 8-32 hours, and more preferably 16-20 hours.

In another preferred embodiment, the step S1 also comprises: crushing the cultured bacteria to obtain the fusion protein.

In another preferred embodiment, the step S1 also comprises: centrifuging to collect the supernatant of the crushed bacteria.

In another preferred embodiment, the step S1 also comprises steps for capturing the fusion protein.

In another preferred embodiment, the step S1 also comprises: subjecting the clarified liquid containing the fusion protein to hydrophobic chromatography capture, thereby obtaining the fusion protein. The mobile phases required for the hydrophobic chromatography capture are: Mobile phase A: 1.7532 g/L of sodium chloride, 0.0605 g/L of sodium dihydrogen phosphate dehydrate and 7.0205 g/L of disodium hydrogen phosphate dodecahydrate; Mobile phase B: pure water.

In another preferred embodiment, in the step S2), the used protease includes: 0.1-5% (preferably 0.5-3%, preferably 1-2%) EK, 0.1-10% (preferably 0.5-5%, preferably 2-4%) kex2, 0.1-10% (preferably 1-5%, preferably 2-4%) CPB, or a combination thereof.

In another preferred embodiment, the enzyme cleavage is performed at 15-35°C, preferably 20-30°C, and more preferably 25-28°C.

In another preferred embodiment, the enzyme cleavage is performed under pH 7-10, preferably pH 7.5-9, and more preferably pH 8-8.5.

In another preferred embodiment, the enzyme cleavage time is 0.5-3 hours, preferably 1-2.5 hours, and preferably 1.5-2 hours.

In another preferred embodiment, in the step S2), after enzymatically cleaving the fusion protein, the clarified liquid containing the target polypeptide GLP-1 analogue is subjected to hydrophobic chromatography capture. The mobile phases: Mobile phase A: 1.7532 g/L of sodium chloride, 0.0605 g/L of sodium dihydrogen phosphate dihydrate, 7.0205 g/L of disodium hydrogen phosphate dodecahydrate; Mobile phase B: pure water.

It should be understood that, within the scope of the present invention, the above technical features of the present invention and the various technical features specifically described below (such as in the examples) can be combined with each other to form new or preferred technical solutions. Due to space limitations, they will not be enumerated one by one here.

### BRIEF DESCRIPTION OF THE DRAWINGS

The following figures are used to illustrate specific embodiments of the present invention and are not intended to limit the scope of the invention as defined by the claims.
Figure 1A shows the combined sequence in which the protein chaperone, protease recognition sequence, and target polypeptide are sequentially connected in the expression vector of the present invention.
Figure 1B shows the map of the expression vector pET32b(+)-kan after resistance replacement.
Figure 2 shows the DNA electropherogram of the expression vector pET32b(+)-kan-GLP110 after enzyme digestion verification. Lanes 1, 2, 3, 4, and 5 show the five selected vectors digested with KpnI and EcoRI double enzymes, and the correct electrophoresis band of about 1.2 kb is obtained in each band. Lane 6 is the DNA marker. This figure is used to indicate the correctness of the construction of the expression vector.

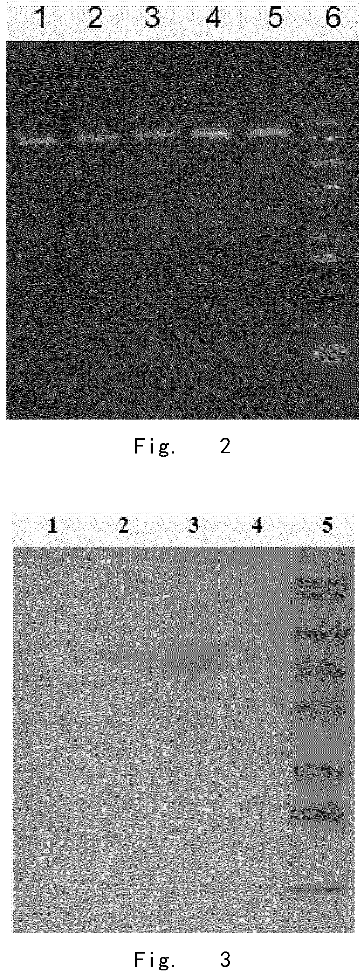

Figure 3 shows the protein electropherogra of different treated samples. Lane 1 is the uninduced whole cell; lane 2 is the whole cell induced by IPTG; lane 3 is the supernatant of the bacterial cells broken by ultrasonication after IPTG induction (concentrated by 1 time); lane 4 is the precipitate of the bacterial cells broken by ultrasonication after IPTG induction; lane 5 is the protein marker. This figure shows that there is almost no band of the target protein (59.6 KD) in the sample of ultrasonic broken precipitate. Therefore, the protein chaperone TR1 can increase the soluble expression of the target protein.
Figure 4 shows the HPLC detection result of the clarified liquid after the cells of the bacteria obtained by small-scale fermentation in SM37 shake flask are broken by a high-pressure homogenizer and clarified by a filter membrane.
Figure 5 shows the HPLC detection result of the fusion protein capture eluent. The results show that hydrophobic chromatography can effectively capture and purify the fusion protein.
Figures 6A, 6B, 6C, and 6D show the HPLC detection results of the enzyme digestion solutions of SM37 fusion protein before enzymatic digestion, after enzymatic digestion for 30 minutes, enzymatic digestion for 1 hour, and enzymatic digestion for 1.5 hours, respectively. It indicates that when the enzymatic digestion lasts for 0.5 hour and 1 hour, the reaction is still continuing. The enzyme digestion reaction is completed in 1.5 hours, and the fusion protein is completely converted into the target protein GLP-1 analogue and protein chaperone.
Figure 7 shows the HPLC detection result of the capture eluent of the target polypeptide obtained by enzymatic digestion of the fusion protein. The result shows that hydrophobic chromatography can effectively capture and purify GLP-1.
Figure 8 shows the mass spectrometry detection result of the target polypeptide.
Figure 9 shows the HPLC detection result of the clarified liquid after the cells of the bacteria obtained by small-scale fermentation in SM38 shake flask are broken by a high-pressure homogenizer and clarified by a filter membrane.
Figure 10A-10D shows the HPLC detection result of the clarified liquid after the cells of the bacteria obtained by small-scale fermentation in SM33-SM36 shake flask are broken by a high-pressure homogenizer and clarified by a filter membrane.
Figure 11A-11D shows the HPLC detection result of the clarified liquid after the cells of the bacteria obtained by small-scale fermentation in SM39-SM42 shake flask are broken by a high-pressure homogenizer and clarified by a filter membrane.
Figure 12 shows the HPLC detection result of the clarified liquid after the cells of the bacteria obtained by small-scale fermentation in SM43 shake flask are broken by a high-pressure homogenizer and clarified by a filter membrane. The yield of fusion protein in SM43 is slightly lower than that in SM37.
Figures 13A-13H show the HPLC detection results of the enzyme digestion solutions of SM43 fusion protein before enzymatic digestion and after enzymatic digestion for 30 minutes, 1 hour, 1.5 hours, 2 hours, 2.5 hours, 3 h and 3.5 h respectively. It indicates that the enzymatic digestion reaction takes 3 to 4 hours, and the fusion protein is completely converted into the target protein GLP-1 analogue and protein chaperone.

### MODES FOR CARRYING OUT THE INVENTION

After extensive and in-depth research, the inventor has developed for the first time a method for producing a polypeptide from a recombinant fusion protein. The method of the present invention obtains a recombinant fusion protein by means of serially combining multiple protease cleavage sites and target polypeptides with the assistance of a specific protein chaperone, and then a polypeptide is prepared by enzymatic digestion. In the present invention, a recombinant strain of fusion protein with high expression level containing serially combining up to 10 polypeptides of GLP-1 analogues (intermediates) is constructed, the fermentation induction expression conditions and enzymatic digestion reaction system are optimized, a capture method for a fusion protein and an intermediate polypeptide is developed, and finally a mass-producible GLP-1 analogue (intermediate) polypeptide is obtained, thus completing the present invention. The target peptide is obtained by enzymatic digestion of high-yield recombinant fusion protein engineering bacteria derived from genetic engineering. This method has low cost and high yield, solving the problems of low reaction efficiency, high impurities, and high cost in existing chemical synthesis methods. On this basis, the present invention is completed.

The present invention has discovered that a fusion protein serially combining multiple polypeptide is almost insoluble without the use of a protein chaperones, making it difficult to use for the production of the target peptide. Introducing protein chaperones into fusion proteins can greatly enhance the solubility and expression levels of highly tandem (e.g. 10) fusion proteins. Meanwhile, the present invention has discovered that introducing specific thioredoxin from *hot spring bacteria* as a protein chaperone can improve the enzymatic cleavage rate of a recombinant fusion protein and further enhance production efficiency.

### Fusion protein

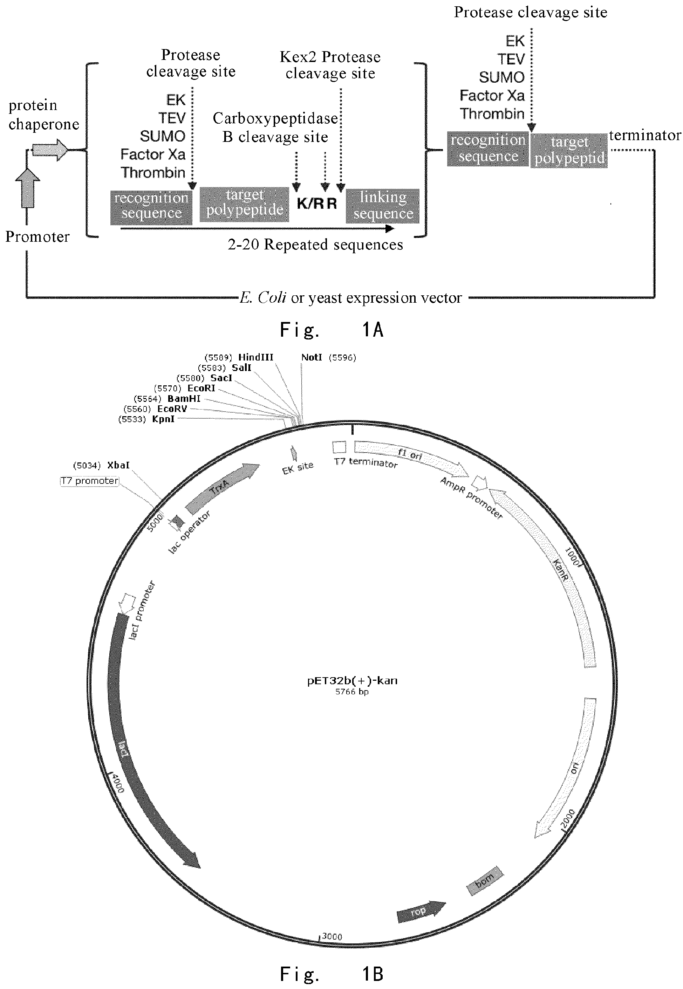

As used herein, the term "recombinant fusion protein" and "fusion protein" can be used interchangeably herein to refer to the recombinant fusion protein of the first aspect of the present invention. The recombinant fusion protein provided in the invention is a serially combining multiple protease cleavage sites and a target polypeptide with the assistance of a specific protein chaperone. As an expression system, it can be used to efficiently express the target polypeptide. The fusion protein of the present invention can optionally serially combine 2-20 target polypeptide sequences, preferably 10 GLP-1 analog peptides. Moreover, the recombinant fusion protein can form independent target peptides through enzymatic cleavage. In a preferred embodiment, the protease recognition sequence includes a combination sequence connected sequentially from the N-terminus to the C-terminus as shown in Figure 1, which contains the structure: {N-terminal protease recognition sequence + target polypeptide + C-terminal protease recognition sequence + linker peptide}₂₋₂₀ + N-terminal protease recognition sequence + target polypeptide.

As used herein, the term "target polypeptide" and "objective polypeptide" can be used interchangeably herein to refer to the polypeptide to be prepared by using the expression system or preparation method of the present invention. According to the teachings of the invention, those skilled in the art can generate various polypeptides as required by utilizing the expression system of the present invention. In specific embodiments, the expression system of the present invention can be used to produce polypeptides with 10 to 100 amino acids and/or an isoelectric point range of 2 to 10, such as glucagon-like peptide, parathyroid hormone, growth factor, collagen, and other medical aesthetic polypeptides

In a preferred embodiment, the expression system of the present invention can be used to produce glucagon-like peptide-1 (GLP-1) or its analogues, glucagon-like peptide (GLP-2) or its analogues, gastric inhibitory peptide (GIP), peptide tyrosine tyrosine (PYY), parathyroid hormone (PTH), growth factor, collagen; preferably glucagon-like peptide-1 (GLP-1) or its analogues, glucagon-like peptide (GLP-2) or its analogues, gastric inhibitory peptide (GIP), peptide tyrosine tyrosine (PYY), growth factor, collagen; more preferably glucagon-like peptide-1 (GLP-1) or its analogues.

As used herein, the term "coding sequence" refer to the sequence encoding the recombinant fusion protein of the first aspect of the present invention, including the amino acid coding sequence and nucleotide coding sequence of the target polypeptide (such as GLP-1 analog (intermediate) polypeptide) expressed in tandem.

The fusion protein of the present invention further comprises a linking peptide for serially combining multiple target polypeptides. The linker peptide used can be a sequence of 2-20 non-acidic amino acids, preferably a flexible linker peptide. The protein sequence of the linking peptide is selected from GGGGS, GSGSG, GSGG, GSGS, or GSG. preferably, GSGSG is used as the connecting peptide.

As used herein, the term "protease recognition sequence" and "protease recognition site" can be used interchangeably herein to refer to sequence contained in the recombinant fusion protein of the first aspect of the present invention that can be recognized and cleaved by a specific protease at its amino-terminal (N-terminal) or carboxy-terminal (C-terminal). In the present invention, an N-terminal protease recognition sequence is connected in series at the N-terminal of the target polypeptide, and this N-terminal protease recognition sequence can be recognized and cleaved at its C-terminal; a C-terminal protease recognition sequence is connected in series at the C-terminal of the target polypeptide, and this C-terminal protease recognition sequence can be recognized and cleaved at its N-terminal to ensure that there are no redundant amino acid residues at both ends of the target polypeptide after cleavage. The protease used can be any protease, provided that it is ensured that the protease will not cleave the target polypeptide.

In one embodiment of the invention, the N-terminal protease recognition sequence used can be the recognition sequence of one or more proteases selected from the group consisting of enteropeptidase, thrombin, SUMO protease, TEV protease and Factor Xa protease; its protein sequence is a series of one or more selected from the group consisting of: SEQ ID NO: 1, SEQ ID NO:2, SEQ ID NO:3, SEQ ID NO:4, SEQ ID NO:5. Preferably, the enteropeptidase protein recognition site is used.

In one embodiment, the C-terminal protease recognition sequence used can be the recognition site of Kex2 protease, carboxypeptidase B (CPB), or its sequence in tandem. In order to ensure that there are no redundant amino acid residues in the polypeptide product after enzymatic digestion, preferably, the carboxypeptidase B (CPB) recognition site is connected in series with the Kex2 protease recognition sequence. The protein sequence can be KR, RR.

### Protein chaperones

As used herein, the term "protein chaperone" refers to a peptide segment that can be used to enhance the solubility and expression efficiency of recombinant fusion proteins, which typically derived from thioredoxin (TRX). Preferably, the protein chaperone is connected to the N-terminus of the fusion protein.

In some embodiments, the amino acid sequence of thioredoxin is selected from the amino acid sequences of SEQ ID NO:8, SEQ ID NO: 10, SEQ ID NO: 12, SEQ ID NO: 14, SEQ ID NO: 16, or SEQ ID NO: 18, or an amino acid sequence having 80%, 85%, 90%, 95%, 96%, 97%, 98%, 99% or higher sequence homology with SEQ ID NO:8, SEQ ID NO: 10, SEQ ID NO: 12, SEQ ID NO: 14, SEQ ID NO: 16, or SEQ ID NO: 18. Preferably, the nucleotide sequence encoding thioredoxin is selected from SEQ ID NO:9, SEQ ID NO: 11, SEQ ID NO: 13, SEQ ID NO: 15, SEQ ID NO: 17, or SEQ ID NO: 19.

The invention promotes the solubility of recombinant fusion proteins by introducing thioredoxin into them. For the convenience of constructing expression vectors, the thioredoxin of the invention can be selected from the inherent thioredoxin on the expression vector (such as TrxA on the pET series expression vector), and from the thioredoxin TR1 TR2, TR3, TR4, TR5 isolated by the inventor from a *hot spring bacterium,* and constructed into an expression vector. In some embodiments, the protein chaperone is the polypeptide of SEQ ID NO:8 (TR1) or the thioredoxin (TrxA) on the pET series expression vector.

The invention also unexpectedly discovered that the efficiency of obtaining the target polypeptide by enzymatic digestion of the recombinant fusion protein can be promoted by selecting a specific thioredoxin. Compared to TrxA, TR1-TR5 from *hot spring bacteria* can achieve higher enzyme digestion efficiency. In some embodiments, the protein chaperone is the polypeptide of SEQ ID NO:8.

### Encoding nucleic acids and combinations thereof

On the basis of the fusion protein of the invention, provided also are isolated polynucleotides or degenerate variants thereof encoding the fusion protein. The polynucleotide of the invention can be DNA or RNA. The DNA include cDNA, genomic DNA, or artificially synthesized DNA. DNA can be single-stranded DNA or double-stranded DNA. DNA can be a coding chain or a non-coding chain. The coding region sequence encoding the mature polypeptide can be the same as or a degenerate variant of the nucleotide sequence encoding the fusion protein in the embodiments of the invention. As used herein, "degenerate variant" of the invention refers to a nucleic acid sequence that encodes the fusion protein in the claims of the invention, but differs from the encoding nucleotide sequence of the fusion protein in the embodiments of the invention.

### Vector, host cell

The encoding polynucleotide sequence can be inserted into a recombinant expression vector or genome. The term "recombinant expression vector" refers to bacterial plasmids, phages, yeast plasmids, plant cell viruses, mammalian cell viruses or other vectors well known in the art. In short, any plasmid and vector can be used as long as it can be replicated and stabilized in the host. An important feature of an expression vector is that it usually contains an origin of replication, a promoter, a marker gene and translation control elements.

The expression vector for expressing the fusion protein of the invention can be either the pQE series expression vector or the pET series expression vector. In a preferred embodiment, the pET-32a/b series expression vectors are used. A typical expression vector such as the pET-32(+)-kan expression vector is obtained by replacing the ampicillin resistance gene on the pET-32b(+) vector with the kanamycin resistance gene. The coding gene of the fusion protein of the invention can be inserted into the XbaI/EcoRI or KpnI/EcoRI enzyme cleavage sites of the pET-32b(+)-kan expression vector.

Those skilled in the art can construct expression vectors containing the DNA sequence encoding the fusion protein of the invention and appropriate transcription/translation control signals use well-known methods, including *in vitro* recombinant DNA technology, DNA synthesis technology, *in vivo* recombination technology, etc. The DNA sequence can be effectively connected to an appropriate promoter in the expression vector to direct mRNA synthesis. The expression vector also includes a ribosome binding site for translation initiation and a transcription terminator.

The host cells described herein include host cells containing the above expression vector or having the coding sequence of the fusion protein of the invention integrated on the genome. Host cells can be prokaryotic cells such as bacterial cells; or lower eukaryotic cells such as yeast cells; or higher eukaryotic cells such as mammalian cells. Representative examples are: *Escherichia coli, Streptomyces; bacterial cells of Salmonella typhimurium;* fungal cells such as *yeast,* plant cells (such as ginseng cells). In a preferred embodiment, the host cells for expressing the fusion protein of the invention is *Escherichia coli,* typically such as *Escherichia coli BL21 (DE3).*

### Preparation method

The present invention provides a method for biosynthesizing a GLP-1 analogue (intermediate) polypeptide. The steps involved are as follows:
(1) Acquiring the coding gene.
(2) Inserting the tandemly expressed coding gene into an expression vector.
(3) Transforming the expression vector into an *Escherichia coli* or *yeast* expression host.
(4) Performing small-scale induction of protein expression by shake flask fermentation.
(5) Collecting and disrupting the bacterial cells.
(6) Capturing the fusion protein.
(7) Cleaving the fusion protein.
(8) Capturing the GLP-1 analogue.

Provided is an improved culture method to increase the expression efficiency of fusion proteins by host cells. The preferred culture method is shake flask fermentation. The specific process is as follows: Inoculate 5 µl of glycerol stock into 5 ml of LB liquid medium containing the corresponding antibiotic. Culture at 37°C and 220 rpm for 16 hours. Inoculate the bacterial solution into 100 ml of LB liquid medium containing 50 µg/ml Kan at a ratio of bacterial solution : medium = 1: 100 (v:v). Culture at 37°C and 220 rpm until the OD600 is approximately 1. Add IPTG to a final concentration of 0.5 mM and induce at 30°C for 20 hours.

The fusion protein of the invention can be expressed intracellularly, on the cell membrane, or secreted outside the cell. If necessary, the host cells can be disrupted by various cell disruption methods to obtain the expressed fusion protein. These methods are well known to those skilled in the art. Examples of these methods include but are not limited to: precipitant treatment (salting-out method), centrifugation, thermal disruption, repeated freezing and thawing, ultrasonic disruption, lysozyme disruption, osmotic disruption, high-pressure homogenization, ultracentrifugation, and combinations thereof.

In a preferred embodiment, high-pressure homogenization and/or ultrasonic disruption are used for cell disruption in the method of the invention.

After the expressed fusion protein is captured and purified, the fusion protein is cleaved into multiple independent target polypeptides by enzymatic digestion. The digestion of fusion protein is preferably performed by co-digestion with three enzymes: enterokinase, carboxypeptidase B, and Kex2 protease.

If necessary, the expressed fusion protein and/or the target polypeptide obtained by enzymatic digestion can be separated and purified by various separation methods utilizing its physical, chemical and other properties. These methods are well known to those skilled in the art. Examples of these methods include but are not limited to: molecular sieve chromatography (gel filtration), adsorption chromatography, ion exchange chromatography, affinity chromatography, low-pressure chromatography, high performance liquid chromatography (HPLC) and other various liquid chromatography techniques and combinations thereof. In a preferred embodiment, hydrophobic chromatography is used in the method of the invention to capture fusion proteins or target polypeptide. Preferably, mobile phase A (sodium chloride, sodium dihydrogen phosphate dihydrate, disodium hydrogen phosphate dodecahydrate) for loading and mobile phase B (pure water) for elution to capture the fusion protein in the hydrophobic interaction chromatography.

### The main advantages of the invention including:

1) A recombinant fusion protein engineering bacterium containing a high-tandem number polypeptides is obtained in the present invention by a thioredoxin fusion expression system. The expressed recombinant fusion protein exhibits advantages, such as a high expression level, few impurity proteins, strong solubility, and no inclusion bodies. Meanwhile, the specific protein chaperone of the invention can increase the enzymatic cleavage rate of the recombinant fusion protein.
2) The invention enables faster cleavage of the fusion protein by proteases by incorporating three protease cleavage sites and flexible linker peptides into the fusion expression vector. The optimized enzymatic cleavage reaction system can complete the enzymatic cleavage of the fusion protein to obtain the target peptide monomer within 1-2 hours.
3) By adopting the improved process for capturing and purifying from fusion protein to GLP-1 analogue, the yield of capture and purification in the invention can reach over 90%, and the purity of the final GLP-1 analogue in the invention can reach over 97%, which is conducive to downstream modification and processing.

The invention is further elaborated below in conjunction with specific embodiments. It should be understood that these embodiments are only used to illustrate the present invention and not to limit the scope of the present invention. Experimental methods without specific conditions specified in the following examples are usually carried out under conventional conditions, such as those described in Sambrook et al., Molecular Cloning: A Laboratory Manual (New York: Cold Spring Harbor Laboratory Press, 1989), or according to the conditions recommended by the manufacturer. Unless otherwise stated, percentages and portions represent percentages by weight and portions by weight.

In the following embodiments, the expression vector pET32b(+) belongs to the pET32 series expression vector and is purchased from Fenghui Biology. Thioredoxin TrxA is present on the pET32 series vector. The expression vector pET32b(+)-kan is obtained by replacing the ampicillin resistance gene on the pET32b(+) vector with the kanamycin resistance gene by the technicians in our laboratory. The vector map is shown in Figure 1B.

### Example 1:

### Acquisition of target gene

The sequence of the tandemly expressed 10-mer GLP-1 analogue polypeptide is: {DDDK + GLP-1 analogue polypeptide sequence + KR + GSGSG}₉ + DDDK + GLP-1 analogue polypeptide sequence. The amino acid sequence is shown as SEQ ID NO:20, and the sequence of the coding gene of the amino acid sequence is shown as SEQ ID NO:21. The codon optimization and synthesis of the coding gene is performed by Suzhou Genewiz Biotechnology Co., Ltd.

### Example 2:

Construction of an expression vector (pET32b(+)-kan) with a kanamycin resistance gene. The pET32 series vectors have an ampicillin resistance gene as the screening marker. Considering that kanamycin is preferred in industrial production, the resistance screening marker on the pET32b(+) vector is changed to a kanamycin resistance gene.

The primers required are as follows:
Forward primer P1: CAGTTTTATTGTtcatgaccaaaatcccttaacgtga (SEQ ID NO.6)
Reverse primer P2: CTAAGAATTAATtcatgagcggatacatatttgaatgtatt (SEQ ID NO.7)
Forward primer P3:
   atccgctcatgaATTAATTCTTAGAAAAACTCATCGAGCATC (SEQ ID NO.30)
Reverse primer P4:
   attttggtcatgaACAATAAAACTGTCTGCTTACATAAACAG (SEQ ID NO.31)

The corresponding vector is constructed through the following steps:
First, the vector was amplified by PCR with commercial pET32b(+) as a template, P1 and P2 as primers. The products were separated by 1.0% agarose gel electrophoresis and a band of about 5 Kb was obtained. The band was cut out of the gel and purified using a gel extraction kit.

The kanamycin resistance gene was amplified by PCR with commercial pET28a(+) as a template, P3 and P4 as primers. The products were separated by 1.0% agarose gel electrophoresis and a band of about 0.9 Kb was obtained. The band was cut out of the gel and purified using a gel extraction kit.

Then pET32b(+)-kan was obtained through seamless ligation according to the system of 50 ng of pET32b(+), 50 ng of kanamycin resistance gene, and 5 µL of 2* seamless cloning reagent, as shown in Figure 1b. And the vector was sequenced and analyzed to ensure that there is no mutation in any vector element.

In the following examples 3-11, during double enzyme digestion with the KpnI/XbaI, the TrxA protein chaperone coding gene carried by the expression vector pET32b(+)-kan itself was removed and replaced with the coding gene of thioredoxin isolated by the inventor from *hot spring bacteria.* In comparative example 1, during double enzyme digestion with the KpnI/XbaI, the TrxA protein chaperone coding gene carried by the expression vector pET32b(+)-kan itself was not affected and remained on the vector. In comparative example 2, during double enzyme digestion with the XbaI/HindIII, the TrxA protein coding gene carried by the expression vector pET32b(+)-kan was removed and a vector without the thioredoxin coding gene was obtained.

### Example 3:

Establishment of an expression vector containing the coding gene of embodiment 1.

In this example, the thioredoxin enzyme isolated from *hot spring bacteria* was used, the amino acid sequence of which is SEQ ID NO: 8 and the encoding gene is SEQ ID NO: 9. The codon optimization and synthesis of the coding gene was performed by Suzhou Genewiz Biotechnology Co., Ltd. The coding gene of thioredoxin was tandemly connected with the coding gene of example 1 for expression of recombinant fusion protein.

The required primers are as follows:
Forward primer P5:
   GCtctagaaataattttgtttaactttaagaaggagatatacatATGAGCCCGATTAAAGAAGA (SEQ ID NO: 32)
Reverse primer P6: GGggtaccAATCTCGCCGCATTCGGCGT (SEQ ID NO: 33)

The expression vector pET32b(+)-kan was double digested with KpnI/XbaI. The digestion reaction system contains 30 µg plasmid (84 µL), 3 µL KpnI, 3 µL Xbal, and 10 µL 10*cutsmart. After digestion at 37°C for 1.5 hours, the expression vector pET32b(+)-kan was separated by 1.0% agarose gel electrophoresis. The approximately 5.2 Kb band of the vector was cut out from the gel and purified using a gel extraction kit.

The target segment was amplified by PCR with synthetic gene SEQ ID NO: 9 as a template, P5 and P6 as primers. The products were separated by 1.0% agarose gel electrophoresis and a band of about 0.5 Kb was obtained. The band was cut out of the gel and recovered using a gel extraction kit. The recovered fragment was double digested with KpnI/XbaI. The digestion reaction system contained 3 µg fragment (43 µL), 1 µL KpnI, 1 µL Xbal, and 5 µL 10*cutsmart. After digestion at 37°C for 1.5 hours, it was separated by 1.0% agarose gel electrophoresis. The approximately 0.5 Kb band was cut out from the gel and recovered using a gel extraction kit. Then, the expression vector was obtained by T4 ligation using a system consisting of 50 ng of vector pET32b(+)-kan double digested with KpnI/XbaI, 50 ng of inserted fragment double digested with KpnI/XbaI, 1 µL of 10*T4 ligase buffer, and 0.5 µL of T4 ligase, and named pET32b(+)-kan-TR1.

The expression vector pET32b(+)-kan-TR1 was double digested with KpnI/EcoRI. The digestion reaction system contained 30 µg plasmid (84 µL), 3 µL KpnI, 3 µL EcoRI, and 10 µL 10*cutsmart. After digestion at 37°C for 1.5 hours, the expression vector pET32b(+)-kan-TR1 was separated by 1.0% agarose gel electrophoresis. The approximately 5.7 Kb band of the vector was cut out from the gel and purified using a gel extraction kit. The recombinant expression vector was obtained by T4 ligation using a system consisting of 50 ng of vector pET32b(+)-kan-TR1 double digested with KpnI/EcoRI, 50 ng of inserted fragment double digested with KpnI/EcoRI, 1 µL of 10*T4 ligase buffer, and 0.5 µL of T4 ligase, and named pET32b(+)-kan-TR1-GLP1¹⁰. Five vectors were picked and verified by double digestion with KpnI/EcoRI, and a band of about 1.2 kb could be digested. The vector was sequenced and analyzed with universal primers T7/T7t to ensure that there is no mutation in the inserted sequence.

### Example 4:

An Escherichia coli genetically engineered expression bacterium was established by transforming the correct recombinant expression vector in embodiment 3 into a host cell.

The host cell was *Escherichia coli BL21 (DE3)* transformed into a competent state. The transformation method was as follows: The competent cells were taken out of the -80°C refrigerator and placed on ice. The expression vector obtained in example 3 was added about 200 ng to 500 ng of melted competent cells. After slowly mixing with a pipette tip, it was incubated on ice for 15 minutes, heated shock at 42°C for 90 seconds and then incubated on ice for 3 minutes. 500 µL of LB culture medium was added and incubated at 37°C and 220 rpm for 45 minutes. Then 50 µL of the aforementioned sample was evenly spread on LB solid medium containing 50 µg/ml. The plate was inverted and cultured overnight to obtain monoclonal colonies. After amplification, it was stored at -80°C with glycerol at a final concentration of 20%. The sequencing analysis confirmed that the obtained sample was a recombinant expression transformant, named SM37.

### Example 5:

### Small-scale shake flask fermentation for production of fusion protein

5 µl of glycerol stock was taken from the glycerol tube of the recombinant expression strain obtained in example 4 that contains the gene encoding the fusion protein containing the 10-mer GLP-1 analogue polypeptide, inoculatd into 5 ml of LB liquid medium containing 50 µg/ml Kan, and cultured at 37°C and 220 rpm for 16 hours. The bacterial solution was inoculated into 100 ml of LB liquid medium containing 50 µg/ml Kan at a ratio of bacterial solution : medium = 1:100 (v:v), and cultured at 37°C and 220 rpm until the OD600 is approximately 1. Isopropyl thiogalactoside (IPTG) was added to a final concentration of 0.5 mM and then induced at 30°C for 20 hours. 20 bottles in parallel were prepared using this method to obtain 1L-2L of fermentation broth. Meanwhile, two bottles without IPTG were used as the control group for non-induced fermentation.

### Example 6:

Bacterial cells from the fermentation broth of example 5 were collected and disrupted.

First, a small amount of bacterial cells were resuspended in 20 mL of 50 mM Tris-HCl buffer at pH 8.0. It was disrupted using ultrasonic wave, and then centrifuged at 4°C and 8000 rpm for 20 minutes. The supernatant and precipitate were separated and subsequently SDS-PAGE electrophoresis was performed. As shown in Figure 3, there was almost no band of the target protein (59.6 KD) in the sample of ultrasonically disrupted precipitate, which means that there is almost no fusion protein in the form of inclusion bodies. It indicates that the protein chaperone TR1 can increase the soluble expression of the protein. There was almost no band of target protein in the group without IPTG induction, indicating that IPTG significantly induces the host cell to express the target protein.

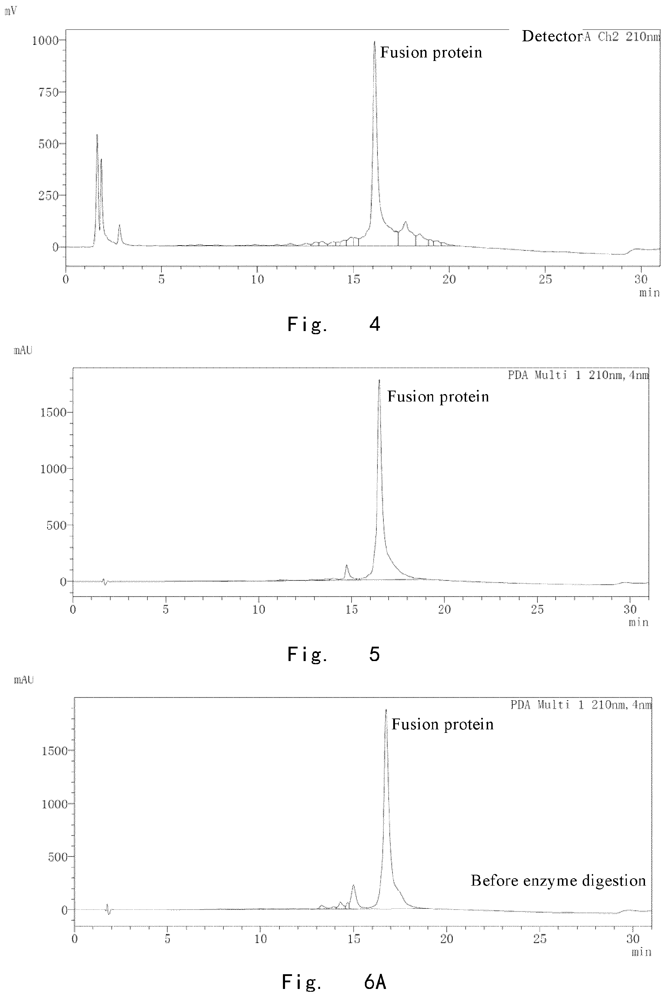

Subsequently, all IPTG-induced bacterial cells were resuspended in 50 mM Tris-HCl buffer at pH 8.0. The concentration of bacterial was controlled at 10%. Then the bacterial cells were disrupted with a high-pressure homogenizer and centrifuged at 4°C and 8000 rpm to collect the supernatant. The obtained supernatant was clarified with a filter membrane of 0.45 µm and sent for HPLC detection. The results are shown in Figure 4.

### Example 7:

Capture of the fusion protein containing the 10-mer GLP-1 analogue polypeptide.

The clarified solution of embodiment 6 containing the fusion protein was captured by hydrophobic interaction chromatography. The required mobile phases were: Mobile phase A: 1.7532 g/L sodium chloride, 0.0605 g/L sodium dihydrogen phosphate dihydrate, 7.0205 g/L disodium hydrogen phosphate dodecahydrate; Mobile phase B: pure water. Mobile phase A was used for loading equilibration, while mobile phase B was used for elution. The result of liquid chromatography of the eluate was shown in Figure 5. This hydrophobic interaction chromatography method can effectively capture and purify the fusion protein, with a yield of more than 90%.

### Example 8:

Enzymatic digestion of the fusion protein containing the 10-mer GLP-1 analogue polypeptide.

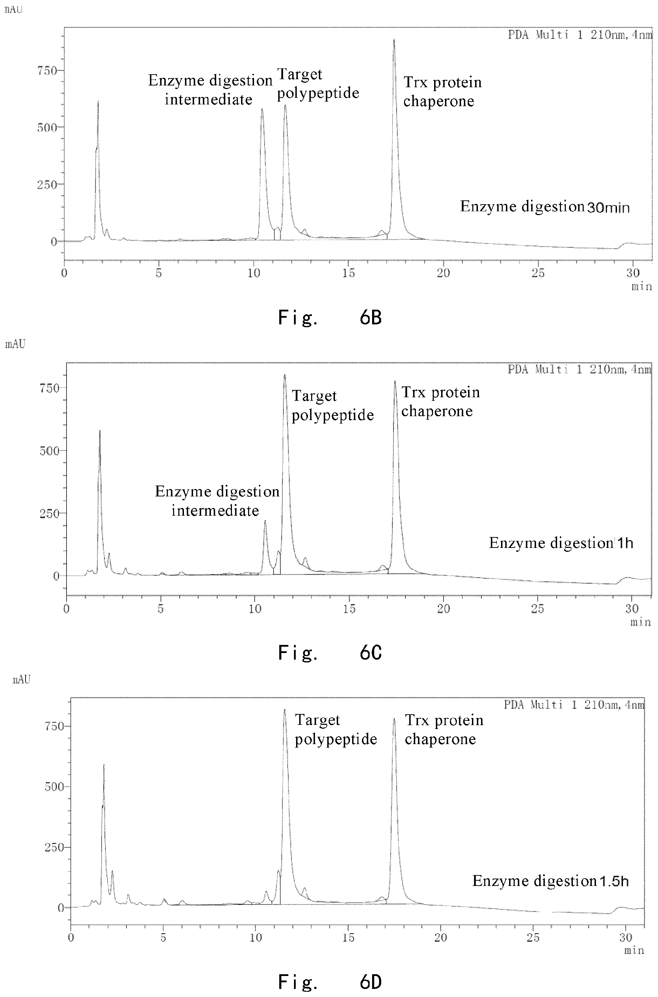

500 mM Tris at pH 8.5 was added to the eluate of embodiment 7 at a volume ratio of 9:1. Then proteases containing 1% EK, 2% kex2, and 2% CPB were added into it. The entire enzymatic digestion process was controlled under buffer conditions of 25°C and pH 8-8.5. During the enzymatic digestion process, sampling and detection were performed once every 30 minutes. As shown in Figure 6A-6D, the detection results of HPLC showed that the reaction was still continuing at 30 minutes of enzymatic digestion. The enzymatic digestion reaction was completed in 1.5 hours, at which point the fusion protein was basically completely converted into the target protein GLP-1 analogue and the thioredoxin chaperone.

### Example 9:

Capture of the target polypeptide GLP-1 analogue.

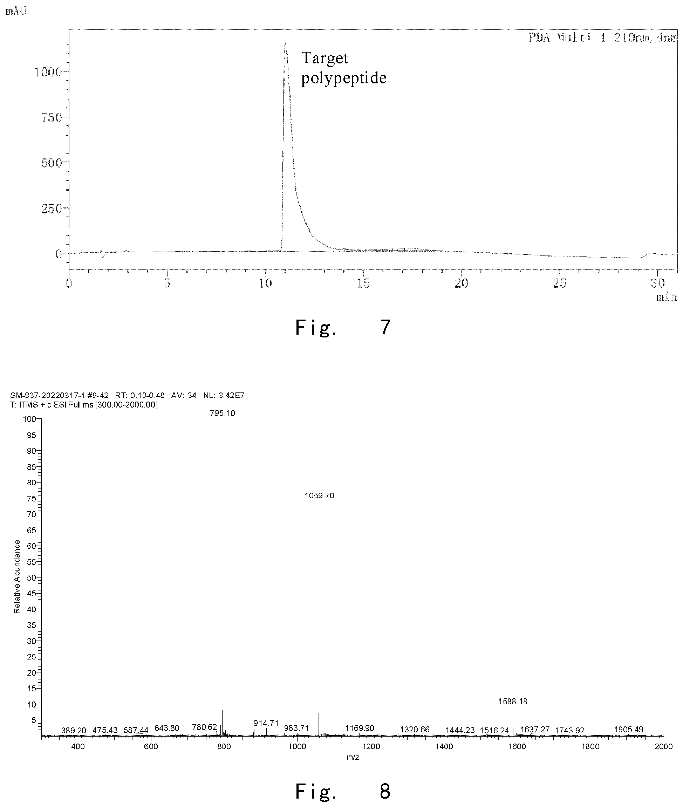

The clarified solution of example 8 containing the target polypeptide GLP-1 analogue was captured by hydrophobic interaction chromatography. The required mobile phases were: Mobile phase A: 1.7532 g/L sodium chloride, 0.0605 g/L sodium dihydrogen phosphate dihydrate, 7.0205 g/L disodium hydrogen phosphate dodecahydrate; Mobile phase B: pure water. Mobile phase A was used for loading equilibration, while mobile phase B was used for elution. The result of liquid chromatography of the eluate was shown in Figure 7 and the mass spectrometry detection result of the purified target polypeptide was shown in Figure 8. It indicated that the hydrophobic interaction chromatography method can effectively capture and purify the GLP-1 analogue, with a yield of more than 90% and a purity of more than 97%.

### Example 10:

A sequence of a tandemly expressed 3-mer GLP-1 analogue polypeptide was designed using the composition form of the recombinant fusion protein coding gene of serially combining a target polypeptide of the invention: {DDDK + GLP-1 analogue polypeptide sequence + KR + GSGSG}₂ + DDDK + GLP-1 analogue polypeptide sequence. The amino acid sequence is shown as SEQ ID NO: 22, and the sequence of the coding gene of the amino acid sequence is shown as SEQ ID NO: 23. The codon optimization and synthesis of the coding gene was performed by Suzhou Genewiz Biotechnology Co., Ltd.

Using the method of embodiment 3, the vector and the inserted fragment were double digested with KpnI and EcoRI and ligated with T4 ligase to construct the recombinant expression vector pET32b(+)-kan-TR1-GLP1³. Then according to the description in embodiment 4, pET32b(+)-kan-TR1-GLP1 is transformed into BL21(DE3) to obtain the recombinant expression transformant named SM-33.

A sequence of a tandemly expressed 4-mer GLP-1 analogue polypeptide was designed using the same method as above: {DDDK + GLP-1 analogue polypeptide sequence + KR + GSGSG}₃ + DDDK + GLP-1 analogue polypeptide sequence. The amino acid sequence is shown as SEQ ID NO: 24, and the sequence of the coding gene of the amino acid sequence is shown as SEQ ID NO: 25. The recombinant expression vector pET32b(+)-kan-TR1-GLP1⁴ was constructed and transformed into BL21(DE3) according to the description in embodiment 4 to obtain the recombinant expression transformant named SM34.

A sequence of a tandemly expressed 5-mer GLP-1 analogue polypeptide was designed using the same method as above: {DDDK + GLP-1 analogue polypeptide sequence + KR + GSGSG}₄ + DDDK + GLP-1 analogue polypeptide sequence. The amino acid sequence is shown as SEQ ID NO: 26, and the sequence of the coding gene of the amino acid sequence is shown as SEQ ID NO: 27. The recombinant expression vector pET32b(+)-kan-TR1-GLP1⁵ was constructed and transformed into BL21(DE3) according to the description in example 4 to obtain the recombinant expression transformant named SM35.

A sequence of a tandemly expressed 6-mer GLP-1 analogue polypeptide was designed using the same method as above: {DDDK + GLP-1 analogue polypeptide sequence + KR + GSGSG}s + DDDK + GLP-1 analogue polypeptide sequence. The amino acid sequence is shown as SEQ ID NO: 28, and the sequence of the coding gene of the amino acid sequence is shown as SEQ ID NO: 29. The recombinant expression vector pET32b(+)-kan-TR1-GLP1⁶ was constructed and transformed into BL21(DE3) according to the description in embodiment 4 to obtain the recombinant expression transformant named SM36.

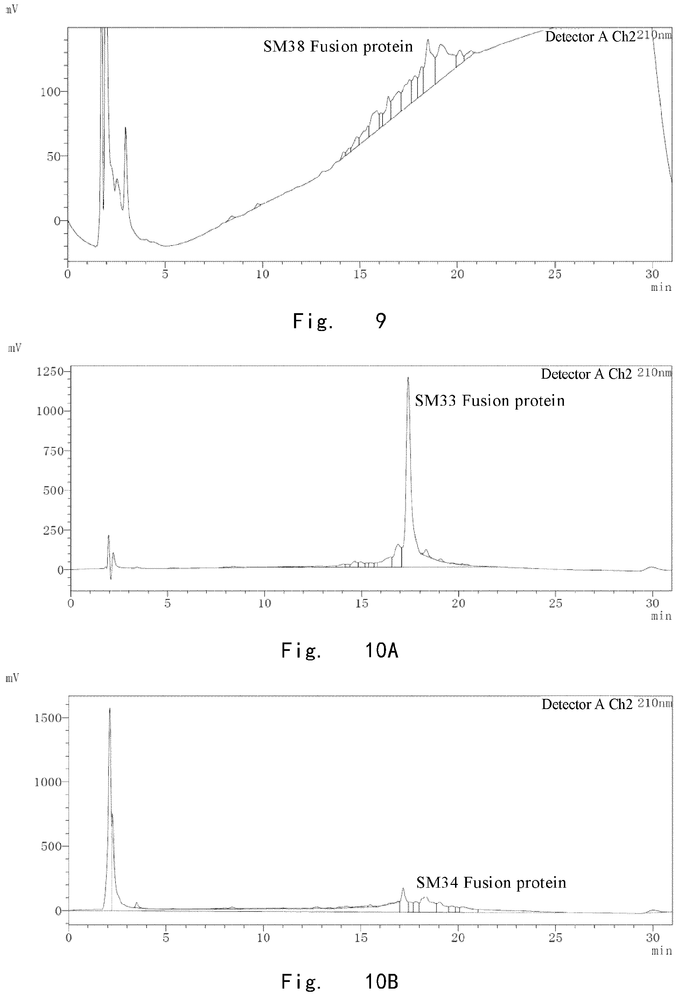

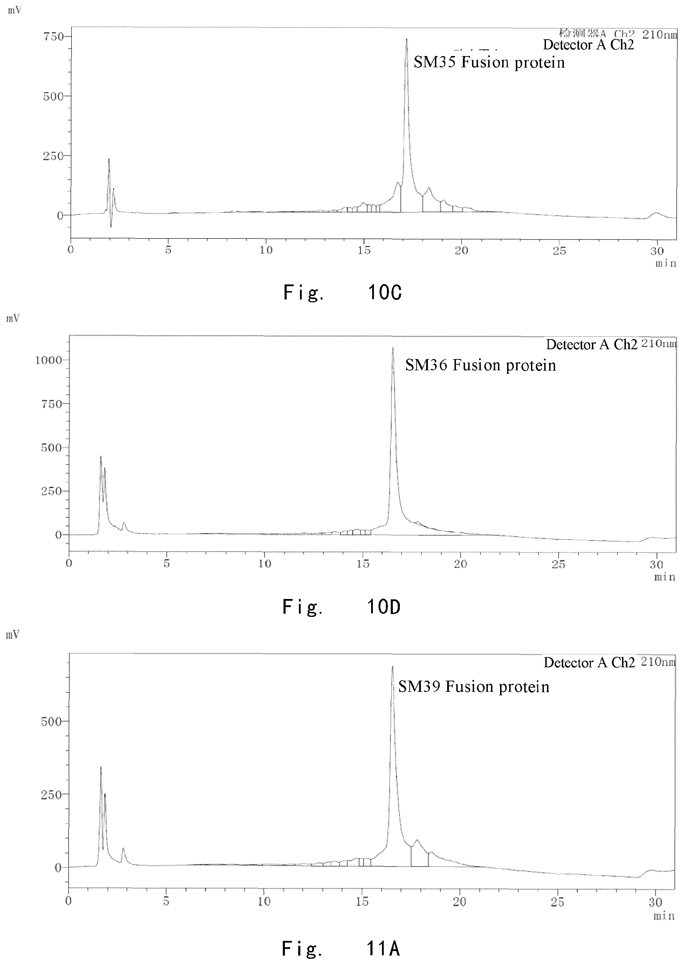

The recombinant expression transformants SM33-SM36 were respectively subjected to small-scale shake flask fermentation to produce fusion proteins. They were induced to express with IPTG at a final concentration of 0.5 mM for 20 hours. Subsequently, the IPTG-induced bacterial cells were resuspended in 50 mM Tris-HCl at pH 8.0. The concentration of bacterial was controlled at 10%. Then the bacterial cells were disrupted with a high-pressure homogenizer and centrifuged at 4°C and 8000 rpm to collect the supernatant. The obtained supernatant was clarified with a filter membrane of 0.45 µm and sent for HPLC detection. The results are shown in Figures 10A, 10B, 10C, and 10D respectively.

The more serial connections there are, the greater the difficulty of expressing fusion proteins. The results of HPLC detection show that the expression levels of fusion proteins are in the order of SM34 < SM35 < SM37 < SM36 < SM33, in which the expression levels of fusion proteins of SM37, SM36, and SM33 are not much different. Since the fusion protein expressed by SM37 is in the form of 10 GLP-1 analogue peptides in tandem, the yield of GLP-1 analogues from the enzymatic digestion of fusion protein expressed by SM37 will be higher than that from the enzymatic digestion of fusion proteins with other numbers of tandem.

### Example 11:

Four other thioredoxin enzymes TR2, TR3, TR4, and TR5 isolated from *hot spring bacteria* were used in this example, the amino acid sequences of which are shown as SEQ ID NO: 10, SEQ ID NO: 12, SEQ ID NO: 14, and SEQ ID NO: 16 respectively. The codon optimization and synthesis of the coding gene was performed by Suzhou Genewiz Biotechnology Co., Ltd. The coding genes are: SEQ ID NO: 11, SEQ ID NO: 13, SEQ ID NO: 15 and SEQ ID NO: 17, respectively. These four thioredoxin enzymes were used to replace the thioredoxin protein chaperone TR1 in pET32b(+)-kan-TR1-GLP1¹⁰ for fusion protein expression. The required primers are as follows:
Forward primer P7:
   GCtctagaaataattttgtttaactttaagaaggagatatacatATGACCCCGGATGCATGGGA (SEQ ID NO.34)
Reverse primer P8: GGggtaccTTCATCTTCTTCACCTTCTTC (SEQ ID NO.35)
Forward primer P9:
   GCtctagaaataattttgtttaactttaagaaggagatatacatATGGCAACCTGTATTGTTCT (SEQ ID NO.36)
Reverse primer P10: GGggtaccCCAATACAGACCACCATACAG (SEQ ID NO.37)
Forward primer P11:
   GCtctagaaataattttgtttaactttaagaaggagatatacatATGCGTGGTTGGCATCGCAT (SEQ ID NO.38)
Reverse primer P12: GGggtaccTTTACCACCAACAACTTCTTT (SEQ ID NO.39)
Forward primer P13:
   GCtctagaaataattttgtttaactttaagaaggagatatacatATGCTGTTTAAACGCCGCGA (SEQ ID NO.40)
Reverse primer P14: GGggtaccCAGATATTCTTCCACAATGCG (SEQ ID NO.41)

The expression vector pET32b(+)-kan-TR1-GLP1¹⁰ was double digested with KpnI/XbaI. The digestion reaction system contains 30 µg plasmid (84 µL), 3 µL KpnI, 3 µL Xbal, and 10 µL 10*cutsmart. After digestion at 37°C for 1.5 hours, the expression vector pET32b(+)-kan-TR1-GLP1¹⁰ was separated by 1.0% agarose gel electrophoresis. The approximately 6.4 Kb band of the vector was cut out from the gel and purified using a gel extraction kit.

The target segment was amplified by PCR with synthetic gene SEQ ID NO: 11, SEQ ID NO: 13, SEQ ID NO: 15, SEQ ID NO: 17 as templates, with P7 and P8, P9 and P10, P11 and P12, and P13 and P14 as primers. The products were separated by 1.0% agarose gel electrophoresis and the bands of about 0.4-0.5 Kb were obtained. The bands were cut out of the gel and recovered using a gel extraction kit. The recovered fragment was double digested with KpnI/XbaI. The digestion reaction system contains 3 µg fragment (43 µL), 1 µL KpnI, 1 µL Xbal, and 5 µL 10*cutsmart. After digestion at 37°C for 1.5 hours, they were separated by 1.0% agarose gel electrophoresis. The approximately 0.4-0.5 Kb bands were cut out from the gel and recovered using a gel extraction kit.

Then, the recombinant expression vector was obtained by T4 ligation using a system consisting of 50 ng of vector pET32b(+)-kan-TR1-GLP1¹⁰ double digested with KpnI/XbaI, 50 ng of inserted fragment, 1 µL of 10*T4 ligase buffer, and 1 µL of T4 ligase, and named pET32b(+)-kan-TR2-GLP1¹⁰, pET32b(+)-kan-TR3-GLP1¹⁰, pET32b(+)-kan-TR4-GLP1¹⁰, pET32b(+)-kan-TR5-GLP1¹⁰. Three vectors were picked for each and verified by double digestion with XbaI/KpnI. The vectors were sequenced and analyzed to ensure that there were no mutations in the inserted fragments. The correct recombinant expression vectors were transformed into the host cell BL21(DE3) to establish genetically engineered Escherichia coli expression bacteria and named SM39, SM40, SM41, and SM42.

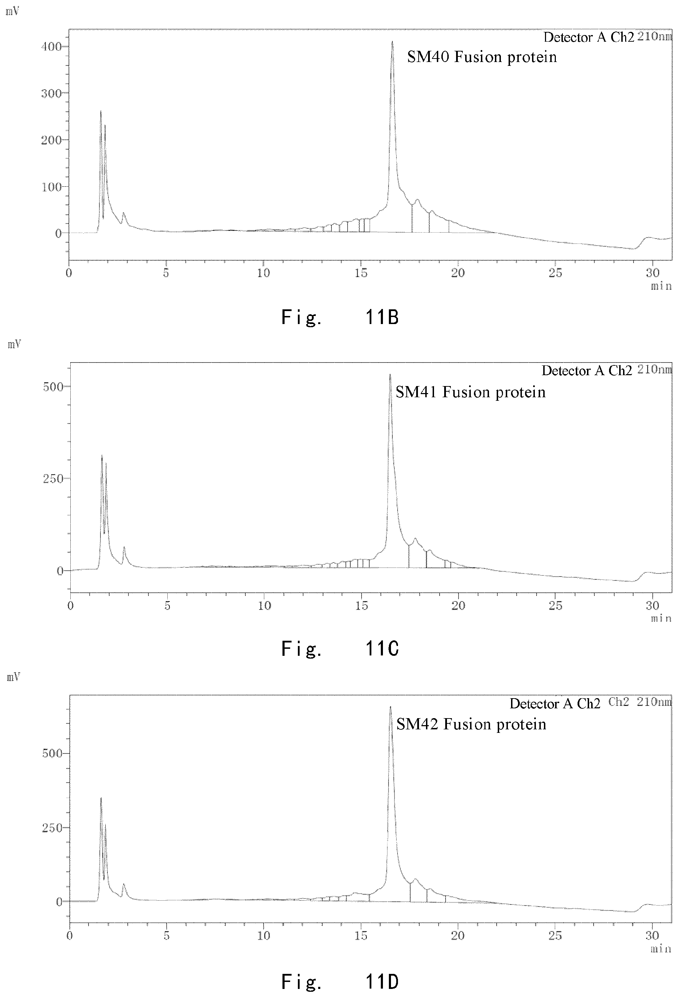

The SM39-SM42 recombinant expression transformants were subjected to small-scale fermentation in shake flasks to produce fusion proteins, followed by the induction of expression for 20 hours using a final concentration of 0.5mM IPTG. The IPTG-induced bacterial cells were resuspended in 50 mM Tris-HCl buffer at pH 8.0. The concentrations of bacterial were controlled at 10%. Then the bacterial cells were disrupted with a high-pressure homogenizer and centrifuged at 4°C and 8000 rpm to collect the supernatant. The obtained supernatants were clarified with a filter membrane of 0.45 µm and sent for HPLC detection. The results are shown in Figure 11A, Figure 11B, Figure 11C and Figure 11D. According to the detection results of HPLC, the fusion proteins of the five expression transformants all have soluble expression, among which SM39 and SM42 have relatively high expression levels. Although the expression levels of fusion proteins corresponding to different thioredoxins are somewhat different, compared with comparative example 2, different thioredoxin chaperones all play certain roles in promoting expression and enhancing solubility.

The above examples have verified that the invention constructs a polypeptide by serially combining multiple protease cleavage sites and polypeptide sequences in a specific order under the assistance of protein chaperones, and provides a method for preparing the fusion polypeptide and small molecule polypeptides by corresponding protease digestion. Based on this method, a recombinant fusion protein strain with high-level expression containing 10 GLP-1 in tandem is constructed. The high-expression fusion protein represents a high-yield GLP-1 analogue. In the invention, *Escherichia coli* is used as the expression host, and the 10 GLP-1 analogues is tandemly expressed, fully exerting the potential of the host. The fusion protein is co-digested by EK, Kex2, and CPB proteases, and the enzymatic digestion reaction can be completed quickly within 1-2 hours. There is no amino acid residue at either the N-terminus or the C-terminus of the obtained GLP-1 analogue. The final purity of the GLP-1 analogue can reach more than 97%, which is conducive to downstream modification and processing.

### Comparative example 1:

Using pET 14a/b series thioredoxin TrxA as a protein chaperone.

The corresponding vector was constructed through the following steps: The expression vector pET32b(+)-kan and the synthesized coding gene were double digested with KpnI/EcoRI respectively to form complementary sticky ends. The digestion reaction system contains 30 µg plasmid (84 µL), 3 µL KpnI, 3 µL EcoRI, and 10 µL 10*cutsmart. After digestion at 37°C for 1.5 hours, it was separated by 1.0% agarose gel electrophoresis. The approximately 5.7 Kb band of vector and the approximately 1.2 Kb band of coding gene were cut out from the gel and purified using a gel extraction kit. The recombinant expression vector was obtained by T4 ligation using a system consisting of 50 ng of vector pET32b(+)-kan, 100 ng of inserted fragment, 1 µL of 10*T4 ligase buffer, and 1 µL of T4 ligase, and named pET32b(+)-kan-TrxA-GLP1¹⁰. Three vectors were picked and verified by double digestion with KpnI/EcoRI, and a correct band of about 1.2 kb could be detected. The vector was sequenced and analyzed with universal primers T7/T7t to ensure that there was no mutation in the inserted sequence. The correct recombinant expression vectors were transformed into the host cell BL21(DE3) to establish genetically engineered *Escherichia coli* expression bacteria and named SM43.

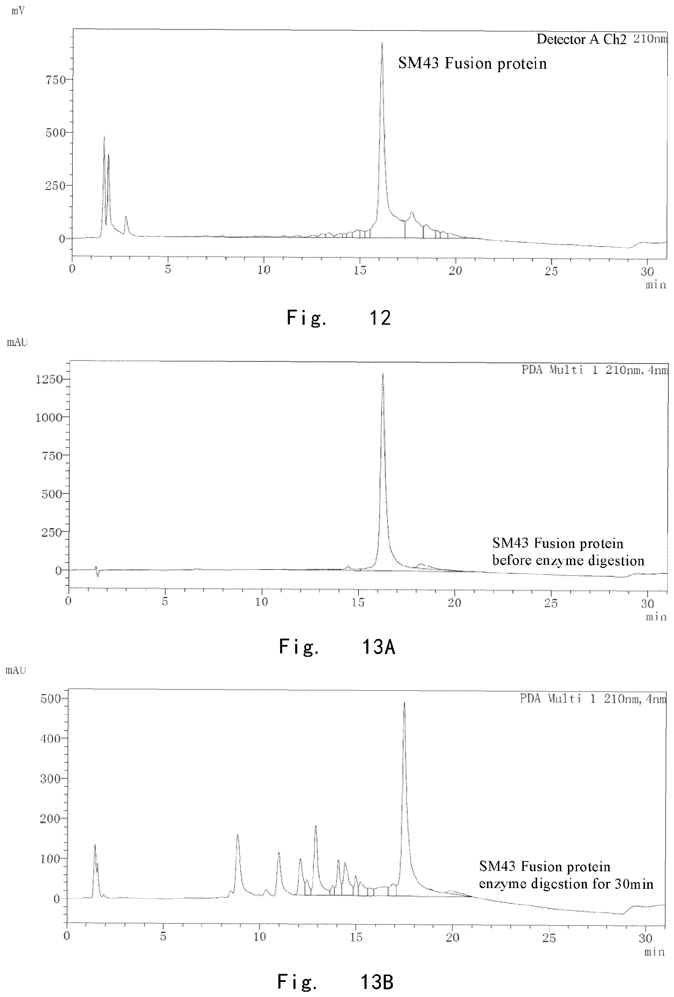

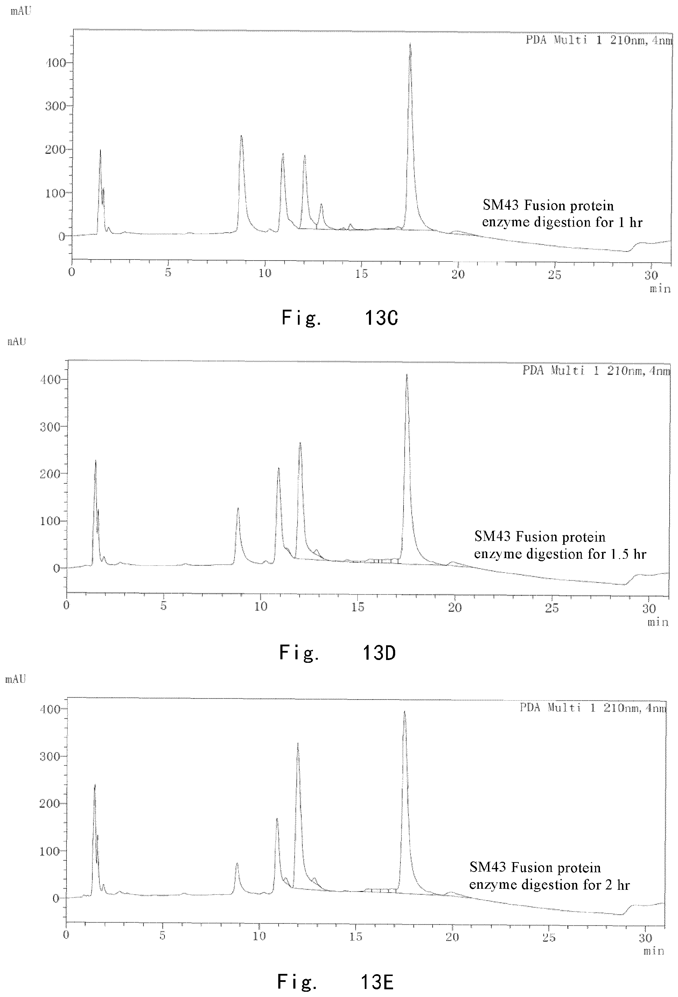

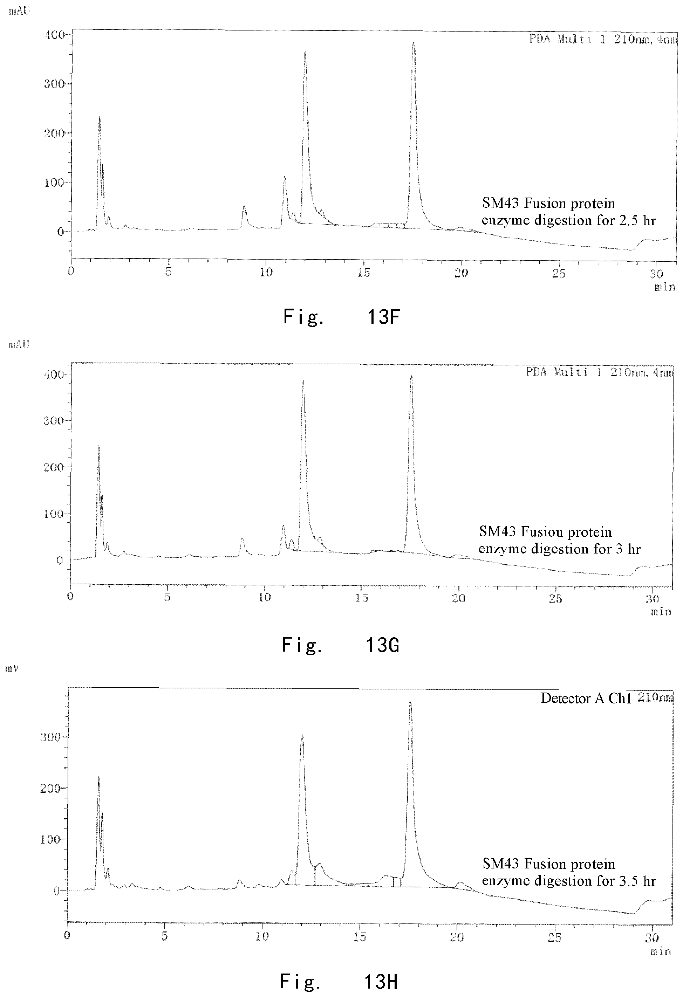

The expression, capture, and fusion enzymatic digestion of the recombinant fusion protein were performed according to the methods described in examples 5-8. The bacterial cells were disrupted with a high-pressure homogenizer and centrifuged to collect the supernatant. The obtained supernatant was clarified with a filter membrane of 0.45 µm and sent for HPLC detection. The results are shown in Figure 12. The expression level of the SM43 fusion protein was slightly lower than that of SM37 fusion protein. Proteases in the same proportion were added after the capture of fusion protein. The sampling and detection were performed once every 30 minutes. The HPLC detection results were shown in Figures 13A-13H. The enzymatic digestion reaction was continuing in 1.5 hours and completed in 3.5 hours. This enzymatic digestion experiment of fusion protein in this comparative example was repeated multiple times and 3-4 hours were necessary to complete the enzymatic digestion.

This comparative example verifies that the enzymatic digestion reaction of the recombinant fusion protein expressed by SM43 requires a longer time, and in the later stage of the enzymatic digestion reaction, the reaction rate slows down even more. This comparative example shows that the protein chaperone TrxA also has the effect of promoting the dissolution and expression of the fusion protein, but the protein structure of the protein chaperone TrxA in the fusion protein may limit the action of proteases on recognition sites, resulting in a slower enzymatic digestion reaction. Using TR1-TR5 isolated by the present inventors from *hot spring bacteria* as protein chaperones can further obtain the unexpected technical effect of increasing the enzymatic digestion rate of the fusion protein.

### Comparative example 2:

An expression vector containing the gene encoded in embodiment 1 was constructed using the vector described in example 2 for a sulfur free oxidoreductor chaperone.

The designed primers are as follows:
Forward primer P15:
   ataacaattcccctctagaaataattttgtttaactttaagaaggagatatacatatgGGTACCGACGACGATGA CAAA (SEQ ID NO.42)
Reverse primer P16:
   tgcggccgcaagcttgtcgacggagctcGAATTCTTATTAGCCGCGGCCACGA (SEQ ID NO.5)

Firstly, the target segment was amplified by PCR with synthetic gene in embodiment 1 as a template, P15 and P16 as primers. The products were separated by 1.0% agarose gel 10 electrophoresis and a band of about 1.2 Kb was obtained. The band was cut out of the gel and purified using a gel extraction kit.

The pET32b(+)-kan was double digested with XbaI/HindIII to form complementary sticky ends. The digestion reaction system contains 30 µg plasmid (84 µL), 3 µL Xbal, 3 µL HindIII, and 10 µL 10*cutsmart. After digestion at 37°C for 1.5 hours, it was separated by 1.0% agarose gel electrophoresis. The approximately 5.2 Kb band was cut out from the gel and purified using a gel extraction kit.

The recombinant expression vector was obtained by seamless ligation using a system of 50 ng of the double-digested and recovered product of pET32b(+)-kan, 100 ng of the PCR amplification product of primers P15 and P16, and 5 µL of 2* seamless cloning reagent, and named pET32b(+)-kan-GLP1¹⁰. Five vectors were selected for double digestion verification with XbaI/HindIII. The vectors were sequenced and analyzed to ensure that there are no mutations in the inserted fragment.

The positive expression vector pET32b(+)-kan-GLP1¹⁰ was transformed into BL21(DE3) according to the method of embodiment 4 to obtain the recombinant transformant named SM38.

Small-scale shake flask induction fermentation was performed according to the method of embodiment 5. The fermentation broth was centrifuged to collect the bacterial cells. Some of the bacterial cells were disrupted by ultrasound and sent for HPLC detection. The results are shown in Figure 9.

The results of HPLC detection show that the soluble expression level of SM38 is extremely low and cannot be used for subsequent operations such as the capture and enzymatic digestion of large amounts of protein for the acquisition of GLP-1 analogue peptides. Since the soluble expression levels of other recombinant transformants with thioredoxin as protein chaperones are relatively high, the thioredoxin chaperone is crucial for the fusion expression of multiple tandem GLP-1 analogues.

The sequences involved in the fusion protein of the invention are listed as follows:
SEQ ID NO: 1
   Name: Intestinal kinase EK cleavage site
   Sequence: DDDDK
SEQ ID NO:2
   Name: SUMO protease cleavage site Sequence: EQIGG
SEQ ID NO:3
   Name: Thrombin enzyme cleavage site Sequence: LVPRGS
SEQ ID NO:4
   Name: TEV enzyme cleavage site Sequence: ENLYFQG(S)
   Name: Factor Xa enzyme cleavage site Sequence: IXGR (X represents E or D)
   Name: Kex2 enzyme cleavage site Sequence: KR/RR
   Name: Carboxypeptidase B cleavage site Sequence: K/R/H
SEQ ID NO:8 
MSPIKEDPINEIDELDSILNTMAQNIIRTSYSRDKTFSNIQTKCCNTVIEGGISI
RSYSEFIQLINSCRVAFVLITTTYCPYCQLFKPVFFRVAREFAGKAVFIEANADY
VPEVAETFNVYSTPTTVIIIDKRPIDAILGYIPYNHFKRYVNDIVSYAECGEI
SEQ ID NO:9 
ATGAGCCCGATTAAAGAAGATCCGATCAACGAAATTGATGAACTGGAT
AGCATTCTGAACACCATGGCCCAGAATATTATCCGTACCTCCTATAGCCGCG
ATAAAACCTTTAGCAACATCCAGACCAAATGCTGCAACACCGTGATCGAGG
GTGGCATTAGCATCCGCAGCTACAGCGAATTTATCCAGCTGATCAACAGCTG
CCGCGTGGCGTTTGTGCTGATCACCACCTACTGCCCGTATTGCCAGCTG
TTTAAACCGGTGTTTTTCCGCGTGGCCCGCGAATTTGCCGGCAAAGCCGTTT
TCATCGAAGCCAACGCCGATTATGTGCCCGAAGTGGCGGAAACCTTTAACG
TGTATAGCACCCCGACCACGGTGATCATTATCGACAAACGCCCGATTGATGC
GATTCTGGGCTACATCCCGTATAACCATTTTAAACGCTATGTGAACGATATT
GTGAGCTACGCCGAATGCGGCGAGATT
SEQ ID NO:10 
MTPDAWDEFDSLWDEYVAKAVEAAKRAGVVLARSYDEYRKAICSKPVA
VVVFTSPTCPACAAYRPIFYEYARRMSQYRGKVAFVEVDSYSAYEAAMEAGV
MATPTTVVYLKCKPVDGFIGLADEETLDEIVRPYITKAVEEGEEDE
SEQ ID NO: 11 
ATGACCCCGGATGCATGGGATGAATTTGATAGCCTGTGGGATGAATAT
GTTGCAAAAGCAGTTGAAGCAGCAAAACGTGCAGGTGTTGTTCTGGCACGT
AGCTATGATGAATATCGTAAAGCAATTTGTAGCAAACCGGTGGCAGTTGTTG
TTTTTACCAGCCCGACCTGTCCGGCATGTGCAGCATATCGTCCGATTTTCTAT
 
GAATATGCACGTCGTATGAGCCAGTATCGTGGTAAAGTTGCATTTGTTGAAG
TTGATAGCTATAGCGCATATGAAGCAGCAATGGAAGCAGGTGTTATGGCAA
CCCCGACCACCGTTGTTTATCTGAAATGTAAACCGGTTGATGGTTTTATTGG
TCTGGCAGATGAAGAAACCCTGGATGAAATTGTTCGTCCGTATATTACCAAA
GCAGTTGAAGAAGGTGAAGAAGATGAA
SEQ ID NO:12 
MATCIVLRLHQGEGLGLLRLTSKSELDRVIRGSRFALVVFTGLACPACEMY
KPVLEKLAELVGKDMPVVEYVVDYDPEPALELGIMGTPTTVVYVDGKPVEGFV
GAVDLPDLLEFLAKVASKSDKQLAEKLEKLAEKVKPLYGGLYW
SEQ ID NO:13 
ATGGCAACCTGTATTGTTCTGCGTCTGCATCAGGGTGAAGGTCTGGGTC
TGCTGCGTCTGACCAGCAAAAGCGAACTGGATCGTGTTATTCGTGGTAGCCG
TTTTGCACTGGTTGTGTTTACCGGTCTGGCATGTCCGGCATGTGAAATGTAT
AAACCGGTTCTGGAAAAACTGGCAGAACTGGTTGGTAAAGATATGCCGGTT
GTTGAATATGTTGTTGATTATGATCCGGAACCGGCACTGGAACTGGGTATTA
TGGGTACACCGACCACCGTTGTTTATGTTGATGGTAAACCGGTTGAAGGTTT
TGTTGGTGCAGTTGATCTGCCGGATCTGCTGGAATTTCTGGCAAAAGTTGCA
AGCAAAAGCGATAAACAGCTGGCAGAAAAACTGGAAAAACTGGCCGAAAA
AGTTAAACCGCTGTATGGTGGTCTGTATTGG
SEQ ID NO:14 
MRGWHRMSSVDEVAAELRKLVEQKIRRIEQDLGDPLYYIEKDFDKVIERY
PVAVVEFSAPWCNPCKAYTPVFRRVARRLIEEYNGKVLFAYLDTDKLPETADR
YNVENIPTTIIFVNGHVADVIMGATTESRLEDKVRSILKEVVGGK
SEQ ID NO:15 
ATGCGTGGTTGGCATCGCATGAGCAGCGTTGATGAAGTTGCAGCAGAA
CTGCGTAAACTGGTTGAACAGAAAATTCGTCGTATTGAACAGGATCTGGGT
GATCCGCTGTATTATATTGAAAAAGATTTTGATAAAGTTATTGAACGTTATC
CGGTTGCAGTTGTTGAATTTAGCGCACCGTGGTGTAATCCGTGTAAAGCATA
TACCCCGGTTTTTCGTCGTGTTGCCCGTCGTCTGATTGAAGAATATAATGGT
AAAGTTCTGTTTGCATATCTGGATACCGATAAACTGCCGGAAACCGCAGATC
GTTATAATGTTGAAAATATTCCGACCACCATTATTTTTGTTAATGGTCATGTT
GCAGATGTTATTATGGGTGCAACCACCGAAAGCCGTCTGGAAGATAAAGTT
CGTAGCATTCTGAAAGAAGTTGTTGGTGGTAAA
SEQ ID NO:16 
MLFKRREKEGNIVEIPCDSKEFKKIIEENKVVVVDFWAKWCFPCLIYARSF
KKAANRLVGRALFAKVNVGECYSLARKYNIFGVPTTMIFVNGKPKRRILGPVS
DKALVRIVEEYL
SEQ ID NO:17 
ATGCTGTTTAAACGCCGCGAAAAAGAAGGCAACATTGTGGAAATCCCG
TGCGATAGCAAAGAATTTAAGAAAATTATCGAAGAGAACAAAGTGGTG
GTGGATTTTTGGGCGAAATGGTGCTTTCCGTGCCTGATTTATGCCCGCAGCT
 
TTAAAAAAGCCGCCAATCGCCTGGTGGGCCGCGCGCTGTTTGCCAAAGTGA
ATGTGGGCGAATGCTATAGCCTGGCCCGCAAATACAACATTTTTGGCGTGCC
GACCACCATGATCTTTGTGAACGGCAAACCGAAACGCCGCATCCTGGGCCC
GGTGAGCGATAAAGCGCTGGTGCGCATTGTGGAAGAATATCTG
SEQ ID NO:18 
MSDKIIHLTDDSFDTDVLKADGAILVDFWAEWCGPCKMIAPILDEIADEYQ
GKLTVAKLNIDQNPGTAPKYGIRGIPTLLLFKNGEVAATKVGALSKGQLKEFLD
ANLA
SEQ ID NO:19 
ATGAGCGATAAAATTATTCACCTGACTGACGACAGTTTTGACACGGAT
GTACTCAAAGCGGACGGGGCGATCCTCGTCGATTTCTGGGCAGAGTGGTGC
GGTCCGTGCAAAATGATCGCCCCGATTCTGGATGAAATCGCTGACGAATATC
AGGGCAAACTGACCGTTGCAAAACTGAACATCGATCAAAACCCTGGCACTG
CGCCGAAATATGGCATCCGTGGTATCCCGACTCTGCTGCTGTTCAAAAACGG
TGAAGTGGCGGCAACCAAAGTGGGTGCACTGTCTAAAGGTCAGTTGAAAGA
GTTCCTCGACGCTAACCTGGCC
SEQ ID NO:20 
DDDDKEGTFTSDVSSYLEGQAAKEFIAWLVRGRGKRGSGSGDDDDKEGT
FTSDVSSYLEGQAAKEFIAWLVRGRGKRGSGSGDDDDKEGTFTSDVSSYLEGQ
AAKEFIAWLVRGRGKRGSGSGDDDDKEGTFTSDVSSYLEGQAAKEFIAWLVRG
RGKRGSGSGDDDDKEGTFTSDVSSYLEGQAAKEFIAWLVRGRGKRGSGSGDD
DDKEGTFTSDVSSYLEGQAAKEFIAWLVRGRGKRGSGSGDDDDKEGTFTSDVS
SYLEGQAAKEFIAWLVRGRGKRGSGSGDDDDKEGTFTSDVSSYLEGQAAKEFI
AWLVRGRGKRGSGSGDDDDKEGTFTSDVSSYLEGQAAKEFIAWLVRGRGKRG
SGSGDDDDKEGTFTSDVSSYLEGQAAKEFIAWLVRGRG*
 *Represents the termination codon (the same below).
SEQ ID NO:21 
GACGACGATGACAAAGAAGGTACGTTTACGAGCGACGTTAGCAGTTAT
CTGGAAGGCCAAGCGGCGAAAGAATTTATCGCCTGGCTGGTGCGCGGTCGT
GGCAAACGTGGCAGCGGTAGCGGCGATGACGATGACAAGGAGGGCACCTTC
ACGAGCGACGTTAGCAGTTATTTAGAAGGCCAAGCCGCCAAAGAGTTTATT
GCGTGGCTGGTTCGCGGTCGTGGCAAACGTGGTAGTGGCAGCGGCGACGAC
GACGACAAAGAAGGCACGTTCACGAGCGACGTTAGCAGCTATCTGGAAGGC
CAAGCGGCCAAAGAGTTTATTGCCTGGCTGGTGCGCGGCCGTGGCAAGCGC
GGCAGCGGCAGTGGTGACGATGACGATAAAGAGGGCACCTTTACGAGCGAT
GTGAGCAGCTATTTAGAGGGTCAAGCGGCGAAGGAGTTTATTGCGTGGTTA
GTGCGCGGCCGCGGCAAACGCGGTAGCGGCAGTGGCGACGACGACGATAA
GGAAGGCACGTTTACGAGCGACGTGAGTAGCTACCTGGAAGGTCAAGCCGC
CAAGGAGTTTATTGCGTGGTTAGTTCGTGGCCGTGGCAAACGCGGTAGTGGC
AGTGGCGACGATGATGACAAAGAGGGCACCTTCACGAGCGATGTGAGTAGC
TATCTGGAAGGCCAAGCGGCCAAAGAATTTATCGCGTGGCTGGTGCGTGGC
CGTGGCAAACGCGGCAGCGGCAGCGGCGATGATGACGATAAAGAGGGCAC
CTTCACGAGCGATGTTAGTAGTTATCTGGAGGGCCAAGCGGCGAAAGAGTT
 
TATTGCCTGGCTGGTGCGTGGTCGTGGCAAACGTGGCAGTGGCAGTGGCGA
TGACGATGACAAAGAGGGCACCTTTACGAGCGATGTGAGTAGCTATCTGGA
AGGCCAAGCGGCGAAAGAGTTTATCGCCTGGCTGGTGCGCGGTCGTGGTAA
GCGCGGCAGCGGCAGTGGCGATGACGACGATAAGGAAGGCACCTTCACGA
GCGATGTGAGCAGTTATCTGGAAGGCCAAGCGGCGAAAGAGTTTATTGCGT
GGTTAGTTCGCGGTCGTGGCAAACGCGGCAGCGGTAGCGGTGATGACGATG
ATAAAGAGGGCACGTTTACGAGCGACGTGAGCAGCTACCTGGAGGGTCAAG
CGGCCAAAGAATTTATTGCGTGGCTGGTTCGTGGCCGCGGCTAA
SEQ ID NO:22 
DDDDKEGTFTSDVSSYLEGQAAKEFIAWLVRGRGKRGSGSGDDDDKEGT
FTSDVSSYLEGQAAKEFIAWLVRGRGKRGSGSGDDDDKEGTFTSDVSSYLEGQ
AAKEFIAWLVRGRG*
SEQ ID NO:23 
GACGACGACGATAAAGAAGGTACTTTTACCTCCGATGTATCTTCCTACC
TGGAGGGCCAGGCTGCTAAAGAATTCATCGCATGGCTGGTGCGTGGTCGTG
GTAAGCGTGGCTCTGGCTCCGGCGACGACGACAAAGAAGGCACGTTTA
CCTCCGACGTTAGCTCTTACCTGGAAGGTCAAGCTGCTAAAGAATTTATCGC
GTGGCTGGTGCGTGGCCGTGGTAAACGTGGTTCCGGCTCTGGTGACGATGAC
GACAAAGAAGGCACCTTCACGTCTGATGTTTCCTCCTATCTGGAAGGCCAGG
CCGCCAAAGAGTTCATCGCGTGGCTGGTTCGTGGTCGTGGTAAACGTGGTTC
CGGTAGCGGCTAA
SEQ ID NO:24 
DDDDKEGTFTSDVSSYLEGQAAKEFIAWLVRGRGKRGSGSGDDDDKEGT
FTSDVSSYLEGQAAKEFIAWLVRGRGKRGSGSGDDDDKEGTFTSDVSSYLEGQ
AAKEFIAWLVRGRGKRGSGSGDDDDKEGTFTSDVSSYLEGQAAKEFIAWLVRG
RG*
SEQ ID NO:25 
GACGATGACGACAAAGAAGGCACCTTCACGAGCGATGTTAGCAGTTAT
CTGGAAGGTCAAGCCGCCAAAGAGTTCATTGCCTGGTTAGTTCGCGGCCGC
GGCAAACGCGGTAGTGGCAGCGGCGACGATGATGACAAAGAAGGCACCTTC
ACGAGCGATGTGAGCAGCTATCTGGAAGGCCAAGCGGCGAAGGAGTTTATC
GCGTGGCTGGTTCGCGGCCGCGGCAAACGCGGCAGCGGCAGTGGCGATGAC
GATGATAAGGAGGGCACGTTTACGAGCGATGTGAGCAGTTACCTGGAAGGC
CAAGCGGCGAAGGAGTTCATTGCGTGGCTGGTGCGTGGCCGTGGTAAGCGT
GGCAGCGGTAGCGGCGACGATGATGACAAAGAGGGCACCTTCACGAGTGAC
GTTAGTAGCTATTTAGAAGGCCAAGCGGCCAAAGAATTTATCGCCTGGTTAG
TGCGCGGTCGTGGCTAA
SEQ ID NO:26 
DDDDKEGTFTSDVSSYLEGQAAKEFIAWLVRGRGKRGSGSGDDDDKEGT
FTSDVSSYLEGQAAKEFIAWLVRGRGKRGSGSGDDDDKEGTFTSDVSSYLEGQ
AAKEFIAWLVRGRGKRGSGSGDDDDKEGTFTSDVSSYLEGQAAKEFIAWLVRG
RGKRGSGSGDDDDKEGTFTSDVSSYLEGQAAKEFIAWLVRGRG*
SEQ ID NO:27 
GACGATGATGACAAAGAAGGCACCTTTACGAGCGACGTGAGCAGTTAC
CTGGAAGGCCAAGCCGCGAAAGAGTTTATTGCCTGGCTGGTGCGCGGCCGT
GGCAAACGTGGCAGTGGCAGCGGCGACGATGACGACAAGGAGGGCACCTTT
ACGAGCGACGTTAGCAGCTACTTAGAAGGCCAAGCGGCCAAGGAATTTATC
GCCTGGCTGGTGCGCGGTCGCGGCAAACGCGGTAGCGGCAGTGGTGATGAC
GATGACAAGGAAGGCACCTTCACGAGCGATGTGAGTAGTTATCTGGAAGGC
CAAGCCGCGAAGGAATTTATCGCGTGGTTAGTTCGCGGCCGTGGTAAACGC
GGCAGCGGCAGCGGCGACGATGACGATAAGGAAGGCACCTTCACGAGCGA
CGTTAGCAGCTACCTGGAAGGCCAAGCGGCCAAAGAGTTCATCGCGTGGCT
GGTGCGTGGTCGCGGTAAACGCGGTAGTGGCAGCGGCGATGACGATGATAA
GGAGGGCACCTTCACGAGCGATGTTAGTAGTTACCTGGAAGGTCAAGCGGC
GAAAGAATTTATTGCCTGGTTAGTGCGTGGCCGCGGTTAA
SEQ ID NO:28 
DDDDKEGTFTSDVSSYLEGQAAKEFIAWLVRGRGKRGSGSGDDDDKEGT
FTSDVSSYLEGQAAKEFIAWLVRGRGKRGSGSGDDDDKEGTFTSDVSSYLEGQ
AAKEFIAWLVRGRGKRGSGSGDDDDKEGTFTSDVSSYLEGQAAKEFIAWLVRG
RGKRGSGSGDDDDKEGTFTSDVSSYLEGQAAKEFIAWLVRGRGKRGSGSGDD
DDKEGTFTSDVSSYLEGQAAKEFIAWLVRGRG*
SEQ ID NO:29

GATGATGACGATAAAGAGGGCACCTTCACGAGCGACGTTAGTAGTTAC
TTAGAGGGTCAAGCGGCGAAGGAATTTATTGCCTGGTTAGTTCGCGGCCGTG
GTAAGCGTGGTAGTGGCAGTGGCGACGATGACGATAAAGAAGGTACGTTCA
CGAGTGACGTTAGTAGCTACCTGGAGGGCCAAGCGGCCAAAGAGTTCATCG
CCTGGCTGGTTCGCGGTCGTGGCAAGCGCGGCAGCGGCAGCGGCGATGACG
ATGATAAAGAAGGCACCTTTACGAGCGACGTGAGCAGCTATCTGGAGGGTC
AAGCGGCGAAAGAATTTATCGCGTGGCTGGTTCGCGGCCGTGGTAAACGTG
GCAGCGGCAGCGGTGACGACGACGATAAAGAAGGCACGTTCACGAGCGAT
GTGAGCAGTTATTTAGAAGGCCAAGCGGCGAAGGAGTTTATCGCGTGGCTG
GTGCGTGGTCGTGGCAAACGCGGCAGCGGTAGTGGCGATGATGACGACAAA
GAAGGCACGTTTACGAGTGATGTGAGTAGTTATTTAGAGGGTCAAGCGGCG
AAAGAGTTCATCGCGTGGCTGGTGCGCGGCCGCGGTAAACGTGGCAGCGGC
AGTGGCGATGACGATGATAAAGAGGGCACCTTTACGAGCGATGTGAGCAGC
TATTTAGAAGGCCAAGCGGCGAAAGAATTTATTGCGTGGCTGGTTCGCGGTC
GTGGCTAA

All references mentioned in the invention are cited as references in this application, as if each reference are cited separately. In addition, it should be understood that after reading the above teaching content of the invention, those skilled in the art can make various modifications or modifications to the present invention, and these equivalent forms also fall within the scope of the claims attached to this application.

## Claims

1. A recombinant fusion protein, wherein the recombinant fusion protein comprises interconnected:
a) a protein chaperone, wherein said protein chaperone is thioredoxin; b) n target polypeptides in tandem;
c) a protease recognition sequence, wherein said protease recognition sequence is used to cleave the recombinant fusion protein into n independent target polypeptides by protease;
n represents an integer of 2-20, preferably 3-15, and more preferably 5-10.

2. The recombinant fusion protein according to claim 1, wherein said thioredoxin is selected from the groups consisting of the following:
(1) an amino acid sequence of SEQ ID NO: 8, SEQ ID NO: 10, SEQ ID NO: 12, SEQ ID NO: 14, SEQ ID NO: 16 or SEQ ID NO: 18;
(2) a polypeptide having at least 80%, at least 85%, or at least 90%, 93%, 95%, 96%, 97%, 98%, 99% amino acid sequence homolog with SEQ ID NO: 8, SEQ ID NO: 10, SEQ ID NO: 12, SEQ ID NO: 14, SEQ ID NO: 16 or SEQ ID NO: 18 and having the function of promoting the solubility of the fusion protein.

3. The recombinant fusion protein according to claim 1, wherein said recombinant fusion protein has the following sequentially connected structures from the N-terminus to the C-terminus:
Protein chaperone-{N-terminal protease recognition sequence-target polypeptide -C-terminal protease recognition sequence-linker peptide}ₙ₋₁-N-terminal protease recognition sequence-target polypeptide.

4. The recombinant fusion protein according to claim 3, wherein said N-terminal protease recognition sequence is a sequence that can be recognized and cleaved by a protease selected from the group consisting of enterokinase, thrombin, SUMO protease, TEV protease, Factor Xa protease, or a combination thereof.

5. The recombinant fusion protein according to claim 3, wherein said C-terminal protease recognition sequence is a sequence that can be recognized and cleaved by a protease selected from the group consisting of Kex2 protease, carboxypeptidase B (CPB), or a dual-enzyme recognition sequence formed by their serially connection.

6. The recombinant fusion protein according to claim 1, wherein said target polypeptide is glucagon-like peptide-1 (GLP-1) or analogues thereof, glucagon-like peptide (GLP-2) or analogues thereof, gastric inhibitory polypeptide (GIP), peptide tyrosine tyrosine (PYY), parathyroid hormone (PTH), growth factors, collagen;
Preferably, glucagon-like peptide-1 (GLP-1) or analogues thereof, glucagon-like peptide (GLP-2) or analogues thereof, gastric inhibitory polypeptide (GIP), peptide tyrosine tyrosine (PYY), growth factors, collagen;
Most preferably, glucagon-like peptide-1 (GLP-1) or analogues thereof.

7. The recombinant fusion protein according to claim 1, wherein said recombinant fusion protein comprises amino acid sequences of SEQ ID NO: 20, SEQ ID NO: 22, SEQ ID NO: 24, SEQ ID NO: 26, SEQ ID NO: 28.

8. The recombinant fusion protein according to claim 7, wherein said recombinant fusion protein comprises amino acid sequences of SEQ ID NO: 20.

9. A polynucleotide encoding the fusion protein according to any one of claims 1-8.

10. A vector comprising the polynucleotide according to claims 9.

11. An engineered host cell comprising the vector according to claim 9, or having the polynucleotide according to claim 9 integrated into its genome.

12. A method for synthesizing a target polypeptide, wherein said method comprising:
S1) Culturing the engineered host cell of the fourth aspect of the present invention under a suitable condition to obtain the fusion protein of the first aspect of the present invention; and
S2) Using an enzyme that specifically cleaves the protease recognition sequence in the fusion protein obtained in step S1) to cleave the fusion protein, thereby obtaining a separate target polypeptide.

13. The method according to claim 12, wherein:
The step S1) includes the steps: inoculating the engineered host cell in a culture medium containing corresponding antibiotics for culturing, and then adding a fermentation inducer for inducing expression, thereby obtaining the fusion protein, and/or
In step S2), the used protease includes: 0.1-5% EK, 0.1-10% kex2, 0.1-10% CPB, or combinations thereof.

14. The method according to claim 12, wherein the step S1 also comprises: crushing the cultured bacteria, centrifuging to collect the supernatant of the crushed bacteria, and subjecting the clarified liquid containing the fusion protein to hydrophobic chromatography capture, thereby obtaining the fusion protein,
The mobile phases required for the hydrophobic chromatography capture are:
Mobile phase A: 1.7532 g/L of sodium chloride, 0.0605 g/L of sodium dihydrogen phosphate dehydrate and 7.0205 g/L of disodium hydrogen phosphate dodecahydrate;
Mobile phase B: pure water.

15. The method according to claim 12, wherein step S2) comprises that after enzymatically cleaving the fusion protein, the clarified liquid containing the target polypeptide GLP-1 analogue is subjected to hydrophobic chromatography capture,
the mobile phases: Mobile phase A: 1.7532 g/L of sodium chloride, 0.0605 g/L of sodium dihydrogen phosphate dihydrate, 7.0205 g/L of disodium hydrogen phosphate dodecahydrate; Mobile phase B: pure water.
